# EUROPEAN PATENT APPLICATION

(11) **EP 2 554 662 A1**
(43) Date of publication of application: **06.02.2013**
(21) Application number: 11176713.3
(22) Date of filing: 05.08.2011
(51) Int. Cl.: C12N 5/0793, A61K 35/28, C12N 5/0789, C12N 5/0735

(54) **Methods of treatment of retinal degeneration diseases**

(71) Applicant: Pia Cosma, M Maria, 08005 Barcelona (ES); Sanges, M Daniela, 08003 Barcelona (ES)
(72) Inventor: Pia Cosma, M Maria, 08005 Barcelona (ES); Sanges, M Daniela, 08003 Barcelona (ES)
(74) Representative: ABG Patentes, S.L.

(57) **Abstract**

The methods comprise administering pluripotent cells to the retina and reprogramming of retinal neurons mediated by cell fusion of said pluripotent cell with said retinal neurons, said reprogramming being mediated by activation of the Wnt/β-catenin pathway.

SEQUENCE LISTING

<110>  Cosma, Maria Pia
       Sanges, Daniela

<120>  METHODS OF TREATMENT OF RETINAL DEGENERATION DISEASES

<130>  P7377EP00

<160>  34

<170>  PatentIn version 3.5

<210>  1
<211>  24
<212>  DNA
<213>  Artificial Sequence

<220>
<223>  Forward primer (Rhodopsin)

<400>  1
gtagatgacc gggttataga tgga                                              24


<210>  2
<211>  21
<212>  DNA
<213>  Artificial Sequence

<220>
<223>  Reverse primer (Rhodopsin)

<400>  2
gcagagaagg aagtcacccg c                                                 21


<210>  3
<211>  19
<212>  DNA
<213>  Artificial Sequence

<220>
<223>  Forward primer (RDS)

<400>  3
cgggactggt tcgagattc                                                    19


<210>  4
<211>  20
<212>  DNA
<213>  Artificial Sequence

<220>
<223>  Reverse primer (RDS)

<400>  4
atccacgttg ctcttgctgc                                                   20


<210>  5
<211>  18
<212>  DNA

<213>  Artificial Sequence

<220>
<223>  Forward primer (Crx)

<400>  5
atccgcagag cgtccact                                                    18


<210>  6
<211>  20
<212>  DNA
<213>  Artificial Sequence

<220>
<223>  Reverse primer (Crx)

<400>  6
cccatactca agtgccccta                                                  20


<210>  7
<211>  20
<212>  DNA
<213>  Artificial Sequence

<220>
<223>  Forward primer (Rx)

<400>  7
gttcgggtcc aggtatggtt                                                  20


<210>  8
<211>  20
<212>  DNA
<213>  Artificial Sequence

<220>
<223>  Reverse primer (Rx)

<400>  8
gcgaggaggg gagaatcctg                                                  20


<210>  9
<211>  18
<212>  DNA
<213>  Artificial Sequence

<220>
<223>  Forward primer (Chx10)

<400>  9
atccgcagag cgtccact                                                    18


<210>  10
<211>  20
<212>  DNA
<213>  Artificial Sequence

<220>
<223>  Reverse primer (Chx10)

<400>  10
cggtcactgg aggaaacatc                                                   20


<210>  11
<211>  14
<212>  DNA
<213>  Artificial Sequence

<220>
<223>  Forward primer (Nestin)

<400>  11
tggaagtggc taca                                                         14


<210>  12
<211>  16
<212>  DNA
<213>  Artificial Sequence

<220>
<223>  Reverse primer (Nestin)

<400>  12
tcagcttggg gtcagg                                                       16


<210>  13
<211>  20
<212>  DNA
<213>  Artificial Sequence

<220>
<223>  Forward primer (Noggin)

<400>  13
cttggatggc ttacacacca                                                   20


<210>  14
<211>  20
<212>  DNA
<213>  Artificial Sequence

<220>
<223>  Reverse primer (Noggin)

<400>  14
tgtggtcaca gaccttctgc                                                   20


<210>  15
<211>  22
<212>  DNA
<213>  Artificial Sequence

<220>
<223>  Forward primer (Otx2)

<400>  15
gagctcagtc gccacctcta ct                                                22


<210>  16

<211>  21
<212>  DNA
<213>  Artificial Sequence

<220>
<223>  Reverse primer (Otx2)

<400>  16
ccgcattgga cgttagaaaa g                                                21


<210>  17
<211>  20
<212>  DNA
<213>  Artificial Sequence

<220>
<223>  Forward primer (hOct4)

<400>  17
tcgagaaccg agtgagaggc                                                  20


<210>  18
<211>  21
<212>  DNA
<213>  Artificial Sequence

<220>
<223>  Reverse primer (hOct4)

<400>  18
cacactcgga ccacatcctt c                                                21


<210>  19
<211>  23
<212>  DNA
<213>  Artificial Sequence

<220>
<223>  Forward primer (hNanog)

<400>  19
ccaacatcct gaacctcagc tac                                              23


<210>  20
<211>  20
<212>  DNA
<213>  Artificial Sequence

<220>
<223>  Reverse primer (hNanog)

<400>  20
gccttctgcg tcacaccatt                                                  20


<210>  21
<211>  19
<212>  DNA
<213>  Artificial Sequence

<220>

<223>  Forward primer (hNestin)

<400>  21
tgtggcccag aggcttctc                                                    19


<210>  22
<211>  18
<212>  DNA
<213>  Artificial Sequence

<220>
<223>  Reverse primer (hNestin)

<400>  22
cagggctggt gagcttgg                                                     18


<210>  23
<211>  19
<212>  DNA
<213>  Artificial Sequence

<220>
<223>  Forward primer (hOtx2)

<400>  23
acccctccgt gggctaccc                                                    19


<210>  24
<211>  19
<212>  DNA
<213>  Artificial Sequence

<220>
<223>  Reverse primer (hOtx2)

<400>  24
cagtgccacc tcctcaggc                                                    19


<210>  25
<211>  20
<212>  DNA
<213>  Artificial Sequence

<220>
<223>  Forward primer (hNoggin)

<400>  25
agcacgagcg cttactgaag                                                   20


<210>  26
<211>  20
<212>  DNA
<213>  Artificial Sequence

<220>
<223>  Reverse primer (hNoggin)

<400>  26
aagctgcgga ggaagttaca                                                   20

<210>  27
<211>  22
<212>  DNA
<213>  Artificial Sequence

<220>
<223>  Forward primer (hCD34)

<400>  27
gttgtcaaga ctcatgaacc ca                                                22


<210>  28
<211>  20
<212>  DNA
<213>  Artificial Sequence

<220>
<223>  Reverse primer (hCD34)

<400>  28
actcggtgcg tctctctagg                                                   20


<210>  29
<211>  20
<212>  DNA
<213>  Artificial Sequence

<220>
<223>  Forward primer (mOct4)

<400>  29
cgtggagact ttgcagcctg                                                   20


<210>  30
<211>  24
<212>  DNA
<213>  Artificial Sequence

<220>
<223>  Reverse primer (mOct4)

<400>  30
gcttggcaaa ctgttctagc tcct                                              24


<210>  31
<211>  20
<212>  DNA
<213>  Artificial Sequence

<220>
<223>  Forward primer (mNanog)

<400>  31
gcgcatttta gcaccccaca                                                   20


<210>  32
<211>  20

<212>  DNA
<213>  Artificial Sequence

<220>
<223>  Reverse primer (mNanog)

<400>  32
gttctaagtc ctaggtttgc                                                    20


<210>  33
<211>  20
<212>  DNA
<213>  Artificial Sequence

<220>
<223>  Forward primer (mCD34)

<400>  33
ctggtacttc cagggatgct                                                    20


<210>  34
<211>  20
<212>  DNA
<213>  Artificial Sequence

<220>
<223>  Reverse primer (mCD34)

<400>  34
tgggtagctc tctgcctgat                                                    20

## Description

### FIELD OF THE INVENTION

This invention relates to the field of cell-based or regenerative therapy for ophthalmic diseases. In particular, the invention provides methods of treatment of retinal degeneration diseases by administering pluripotent cells to the retina and reprogramming of retinal neurons mediated by cell fusion of said pluripotent cell with said retinal neurons, said reprogramming being mediated by activation of the Wnt/β-catenin pathway.

### BACKGROUND OF THE INVENTION

The retina is a specialized light-sensitive tissue at the back of the eye that contains photoreceptor cells (rods and cones) and neurons connected to a neural network for the processing of visual information. The rods function in conditions of low illumination whereas cones are responsible for color vision and all visual tasks that require high resolution (e.g. reading). The rods are mostly located away from the center of the eye in the retinal periphery. The highest concentration of cones is found at the center of the retina, the macula, which is necessary for visual acuity. For support of its metabolic functions, the retina is dependent on cells of the adjacent retinal pigment epithelium (RPE).

Retinal degeneration is the deterioration of the retina caused by the progressive and eventual death of the retinal or retinal pigment ephitelium (RPE) cells. There are several reasons for retinal degeneration, including artery or vein occlusion, diabetic retinopathy, retrolental fibroplasia/retinopathy of prematurity, or disease (usually hereditary). These may present in many different ways such as impaired vision, night blindness, retinal detachment, light sensitivity, tunnel vision, and loss of peripheral vision to total loss of vision. Retinal degeneration is found in many different forms of retinal diseases including retinitis pigmentosa, age-related macular degeneration (AMD), diabetic retinopathy, cataracts, and glaucoma.

Retinitis pigmentosa (RP) is the most common retinal degeneration with a prevalence of approximately 1 in 3,000 to 1 in 5,000 individuals, affecting approximately 1.5 million people worldwide. RP is a heterogeneous family of inherited retinal disorders characterized by progressive degeneration of the photoreceptors with subsequent degeneration of RPE. It is the most common inherited retinal degeneration and is characterized by pigment deposits predominantly in the peripheral retina and by a relative sparing of the central retina. The typical manifestations are present between adolescence and early adulthood and lead to devastating visual loss with a high probability. In most of the cases of RP, there is primary degeneration of photoreceptor rods, with secondary degeneration of cones. RP is a long-lasting disease that usually evolves over several decades, initially presented as night blindness, and later in life as visual impairment in diurnal conditions. Currently, there is no therapy that stops the evolution of retinal degeneration or restores vision. There are few treatment options such as light avoidance and/or the use of low-vision aids to slow down the progression ofRP. Some practitioners also consider vitamin A as a possible treatment option to slow down the progression of RP.

Effective treatment for retinal degeneration has been widely investigated. The field of stem cell-based therapy holds great potential for the treatment of retinal degenerative diseases as many studies in animal models suggest that stem cells have the capacity to regenerate lost photoreceptors and retinal neurons and improve vision. To date, these cells include retinal progenitor cells, embryonic stem cells, bone marrow-derived stem cells, and induced pluripotent stem cells.

Retinal progenitor cells (RPC) are derived from fetal or neonatal retinas, and comprise an immature cell population that is responsible for generation of all retinal cells during embryonic development. RPC can proliferate and generate new neurons and specialized retinal support cells in vitro, and can also migrate into all retinal layers and develop morphological characteristics of various retinal cell types in vivo (MacLaren et al., 2006, Nature 444:203-7). These results support the hypothesis that RPC transplants are a potential treatment for retinal degenerative diseases.

Embryonic stem cells (ESC) are derived from the inner cell mass of blastocyst-stage embryos, with self-renewal capabilities as well as the ability to differentiate into all adult cell types, including photoreceptor progenitors, photoreceptor, or RPE in mice and humans (Lamba et al., 2006, PNAS USA 103:12769-74; Osakada et al., 2008, Nat Biotechnol 26:215-224). Lamba *et al.* showed that transplantation of retinal cells derived from human ESC into the subretinal space of adult Crx(^{-/-}) mice promoted the differentiation of hESC-derived retinal cells into functional photoreceptors, and the procedure improved light responses in these animals (Lamba et al., 2009, Cell Stem Cell 4:73-9). Although ESC are promising in retinal replacement therapies, there remain ethical and immune rejection issues to be considered, and ESC have also been associated with teratoma formation.

The bone marrow harbors at least two distinct stem cell populations: mesenchimal stem cells (MSC) and hematopoietic stem cells (HSC). MSC can be induced into cells expressing photoreceptor lineage-specific markers in vitro using activin A, taurine, and epidermal growth factor (Kicic et al., 2003, J Neurosci 23:7742-9). In addition, an *in vivo* animal model demonstrated that MSC injected into the subretinal space can slow down retinal cell degeneration and integrate into the retina and differentiate into photoreceptors in Royal College of Surgeons (RCS) rats (Kicic et al., 2003, J Neurosci 23:7742-9; Inoue et al., 2007, Exp Eye Res 85:234-41).

Otani *et al.* have reported that intravitreally injected, lineage-negative (Lin⁻) hematopoietic stem cells (HSC) can rescue retinal degeneration in *rd1* and *rd10* mice (Otani et al., 2004, J Clin Invest 114:755-7; US 2008/0317721; US 2010/0303768). However, the transplanted retinas were formed of nearly only cones, and the electroretinogram responses were severely abnormal and comparable to untreated animals. There was a limitation in that intravitreally injected Lin⁻ HSC were effectively incorporated into the retina only during an early, postnatal developmental stage but not in adult mice, only targeting activated astrocytes that are observed in neonatal mice or in an injury induced model in the adult (Otani et al., 2002, Nat Med 8:1004-10; Otani et al., 2004, J Clin Invest 114:755-7; Sasahara et al., 2004, Am J Pathol 172:1693-703).

Induced pluripotent stem cells (iPS) derived from adult tissues are pluripotent ESC-like cells reprogrammed *in vitro* from terminally differentiated somatic cell by retroviral transduction of four transcription factors: Oct3/4, Sox2, Klf4 and c-Myc. It has been reported that human iPS have a similar potential of ESC to mimic normal retinogenesis (Meyer et al., 2009, PNAS USA 106:16698-703). However, major issues include reducing the risk of viral integrations and oncogene expression for generation of iPS. These limitation may be overcome using alternative methods to obtain iPS such as activation of signaling pathways, including the Wnt/β-catenin, MAPK/ERK, TGF-β and PI3K/AKT signaling pathways (WO 2009/101084; Sanges & Cosma, 2010, Int J Dev Biol 54:1575-87).

Therefore, there is the need to provide an effective method for treating retinal degenerative diseases.

### SUMMARY OF THE INVENTION

Inventors have now found that retinal regeneration can be achieved by implanting pluripotent cells into the retina of a subject which fuse with retinal neurons to form hybrid cells which reactivate neuronal precursor markers, proliferate, de-differentiate and finally differentiate into terminally differentiated retinal neurons of interest, e.g., photoreceptor cells, which can regenerate the damaged retinal tissue. The activation of the Wnt/β-catenin signalling pathway is essential to induce de-differentiation of said hybrid cells and final re-differentiation in the retinal neurons of interest. In an embodiment, activation of the Wnt/β-catenin signalling pathway is, at least partially, provided by the implanted pluripotent cells (which have been treated with a Wnt/β-catenin pathway activator, or overexpress a Wnt/β-catenin pathway activator), whereas in another embodiment, activation of the Wnt/β-catenin signalling pathway is only provided as a result of administering a Wnt/β-catenin signalling pathway activator to the subject to be treated or as a consequence of a retinal damage or injury, as occurs in, for example, retinal degeneration diseases (e.g., Retinitis Pigmentosa). The newborn retinal neurons fully regenerate the retina in the transplanted mammalian, with some rescue of functional vision. Histological analysis shows that said regenerated retinas are indistinguishable from retinas of wild-type mammalians two months after transplantation. These data show that pluripotent cell fusion-mediated regeneration is a very efficient process in mammalian retina, and that it can be triggered by activation of Wnt signaling in the transplanted cells, and that *in vivo* reprogramming of terminally differentiated retinal neurons is a possible mechanism of tissue regeneration. Consequently, these teachings can be applied to treat diseases wherein retina is degenerated.

### BRIEF DESCRIPTION OF THE DRAWINGS

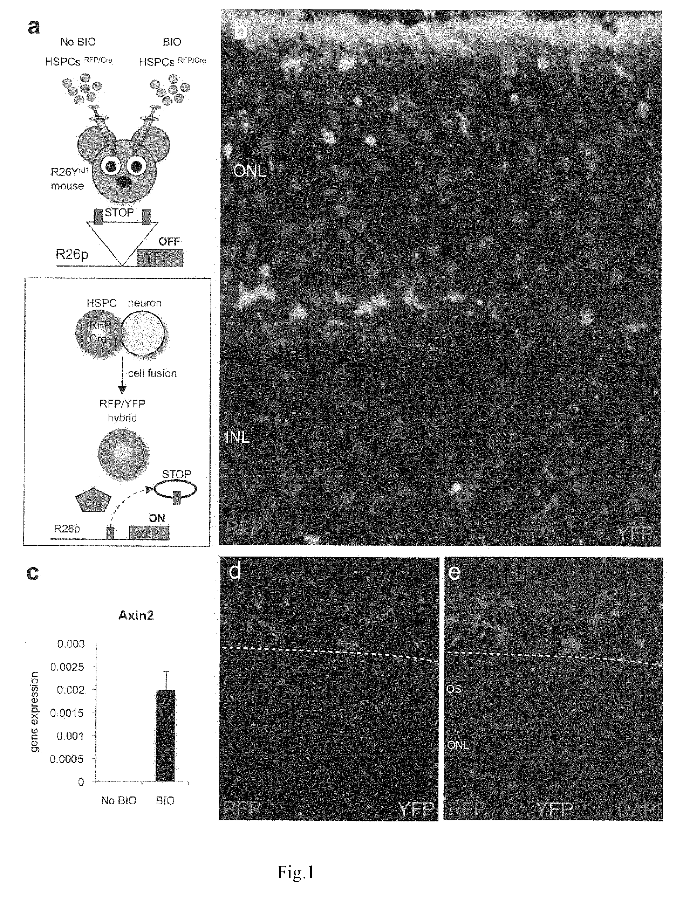

Figure 1. Cell fusion controls. (a) Schematic representation of the experiment plan. *In-vivo* cell fusion between HSPCs^{RFP/Cre} with retinal neurons of LoxP-STOP-LoxP-YFP mice (R26Y) leads to excision of a floxed stop codon in the retinal neurons, and in turn, to expression of YFP. The resulting hybrids express both RFP and YFP. (b) Representative fluorescence micrographs of R26Y*^{rd1}* retinas 24 h after subretinal transplantation of BIO-treated HSPCs^{CRE/RFP}. YFP positive cells represent hybrids derived from cell fusion of HSPCs with R26Y*^{rd1}* retinal cells. (c) RT-PCR analysis of the target gene Axin2 shows β-catenin signalling activation in BIO-treated HSPCs. (d-e) Representative fluorescence micrographs of p 10 wild-type R26Y retinas 24 h after subretinal transplantation of BIO-treated HSPCs^{CRE/RFP}. No YFP-positive cells (green) were detected. Nuclei were counterstained with DAPI in (e). Dotted lines show the final part of the retinal tissue. OS: outer segment; ONL: outer nuclear layer; INL: inner nuclear layer.

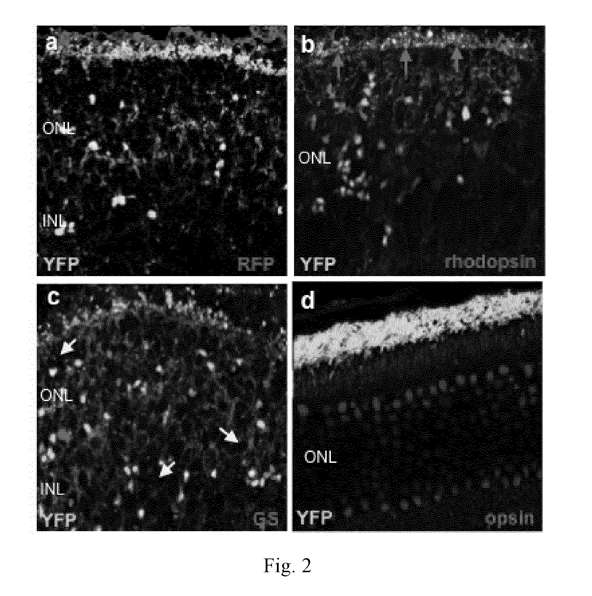

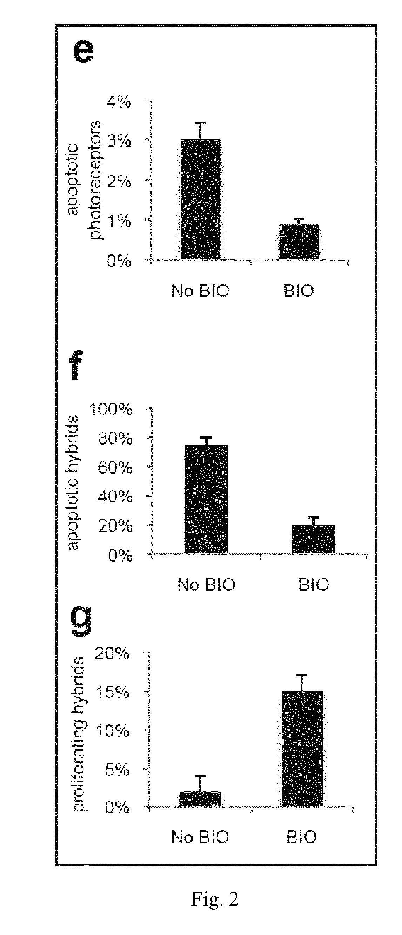

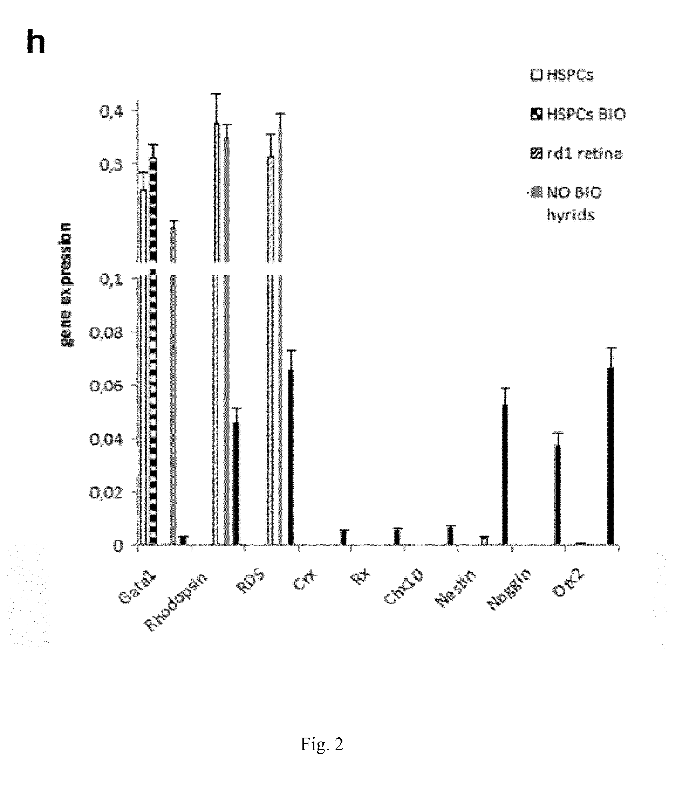

**Figure 2****.** Transplanted HSPCs fuse and induce de-differentiation of *rdl*mouse retinal cells upon Wnt signalling activation. (a-d) Representative fluorescence micrographs of R26Y*^{rd1}*ouse retinas 24h after subretinal transplantation of HSPCs^{CRE/RFP}. Double-positive RFP/YFP (red/green) hybrids following cell fusion of HSPCs (red) with *rd1* retinal cells were detected in the ONL, and a few in the INL These YFP-positive hybrids (YFP, green) are also positive for markers to rod (rhodopsin; red in b) and Müller (glutamine synthetase; red in c) cells, but not to cones (d). (e-g) Quantification of apoptotic photoreceptors (e) and apoptotic (f) and proliferating (g) hybrids 24h after transplantation of non-BIO-treated (No BIO) and BIO-treated (BIO) HSPCs^{CRE} in p10 R26Y^{rd1} eyes. Numbers were calculated as the percentage of TUNEL positive photoreceptors with respect to total photoreceptor nuclei (e) or as the percentage of Annexin V (f) or Ki67 (g) positive cells with respect to the total numbers of YFP positive hybrid cells. (h) Real-time PCR of genes (as indicated) expressed in the HSPCs and retinal and hybrid cells (as indicated). ONL: outer nuclear layer; INL: inner nuclear layer.

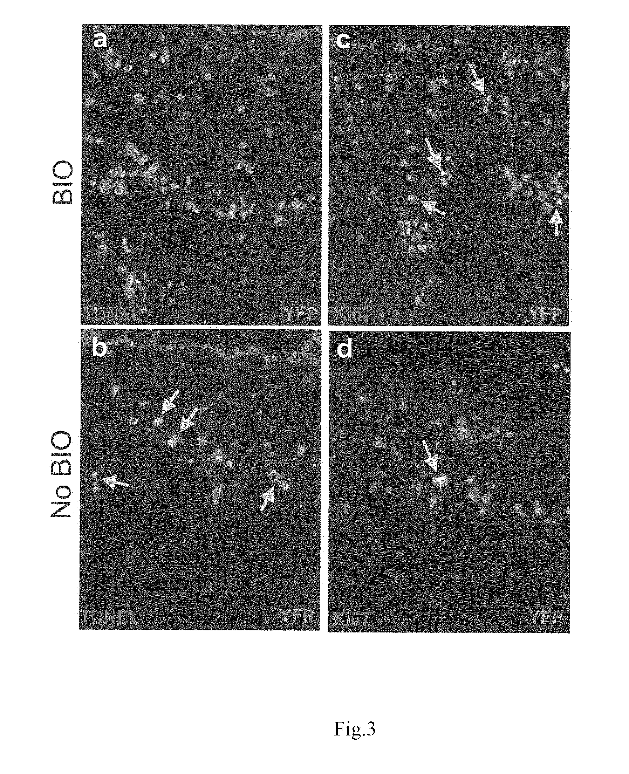

**Figure 3****.** Proliferation and cell-death analysis of de-differentiated hybrids. Representative immunofluorescence staining of Annexin V (a, b) and Ki67 (c, d) on retinal sections of R26Y*^{rd1}* mice analysed 24 h after transplantation at p10 with BIO-treated HSPCs^{CRE} (BIO; a, c) or non-treated HSPCs^{CRE} (No BIO; b, d). YFP fluorescence (green) localises hybrids obtained after fusion. Nuclei were counterstained with DAPI (blue). Yellow arrows indicate apoptotic (b) or proliferating hybrids (c-d).

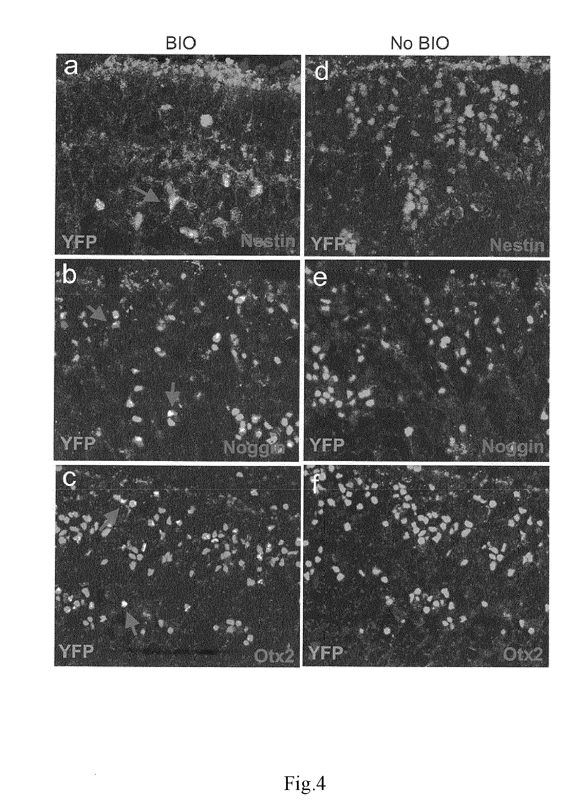

**Figure 4****.** Immunofluorescence analysis of expression of precursor markers in de-differentiated hybrids. Representative immunofluorescence staining of Nestin (a, d, red), Noggin (b, e, red) and Otx2 (c, f, red) in retinal sections from R26Y*^{rd1}* mice 24 h after transplantation at p10 of BIO-treated HSPCs^{CRE} (BIO; a-c) or untreated cells (No BIO; d-f). YFP hybrids (green) obtained after fusion were positive for these markers only following BIO-treatment (a-c, yellow arrows).

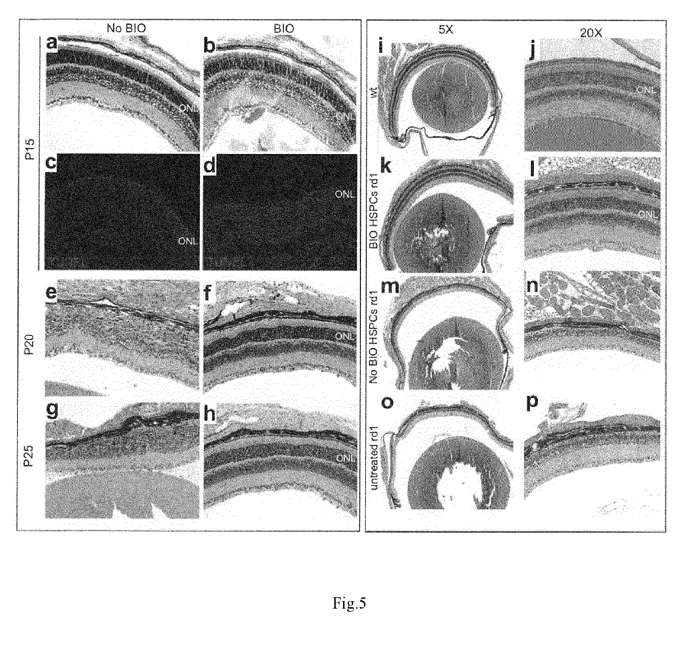

**Figure 5****.** Histological analysis time course of retinal regeneration in *rd1* mice. (a-h) Representative H&E staining (a, b, e-h) and TUNEL staining (c, d; red) of retinal sections of R26Y^{rd1} mice transplanted at p10 with untreated (a, c, e, g) or BIO-treated (b, d, f, h) HSPCs^{RFP/}CRE and analysed 5 (p15; a-d), 10 (p20; e, f) and 15 days (p25; g, h) after transplantation. (i-p) Representative H&E staining of wild-type (i, j) and *rd1* mice (k-p) without transplantation (i, j, o, p) or transplanted at p10 (k-n) with untreated (m, n) or BIO-treated (k-1) HSPCs (m-n), all analysed at p60. Magnification: 20× α-h, j, 1, n, p; 5 × i, k, m, o. ONL: outer nuclear layer.

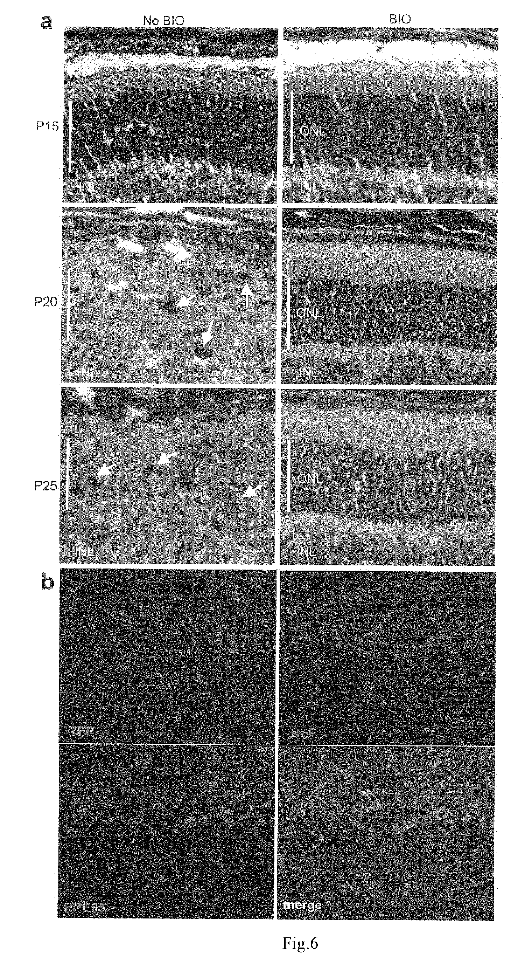

**Figure 6****.** Histological analysis time course of transplanted R26Y*^{rd1}*eyes. (a-h) Representative H&E staining (a, b, e-h) and TUNEL staining (c, d; red) of retinal sections of R26Y^{rd1} mice transplanted at p10 with untreated (a, c, e, g) or BIO-treated (b, d, f, h) HSPCs^{RFP/CRE} and analysed 5 (p15; a-d), 10 (p20; e, f) and 15 days (p25; g, h) after transplantation. (i-p) Representative H&E staining of wild-type (i, j) and *rd1* mice (k-p) without transplantation (i, j, o, p) or transplanted at p10 (k-n) with untreated (m, n) or BIO-treated (k-1) HSPCs (m-n), all analysed at p60. Magnification: 20x, α-h, j, 1, n, p; 5 x i, k, m, o. ONL: outer nuclear layer; INL: inner nuclear layer.

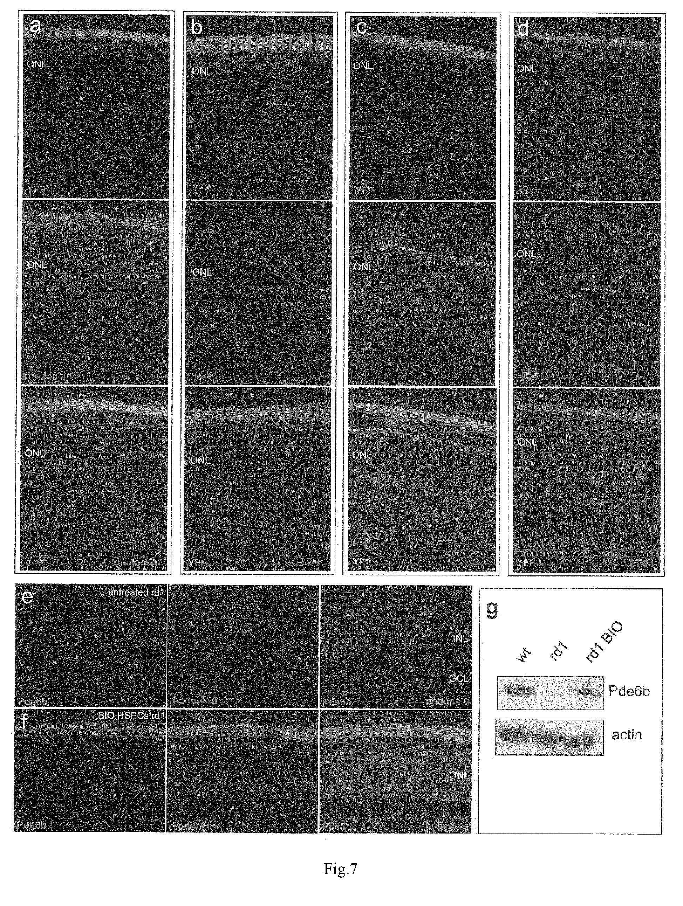

**Figure 7****.** Analysis of hybrid differentiation at p60. (a-f) Representative immunofluorescence staining of retinal sections of R26Y^{rd1} mice without transplantation (e) and transplanted at p10 with BIO-treated HSPCs (a-d, f) and analysed at p60. (a-d) YFP-positive hybrids (green) are positive for rhodopsin (a, red) but not for cone opsin (b, red), glutamine synthetase (c, red), and CD31 (d, red). Bottom images: merges of red and green, with nuclei also counterstained with DAPI (blue). (e-f) Rhodopsin (red), Pde6b (magenta) and counterstained nuclei with DAPI (blue). (g) Western blotting of Pde6b protein expression in the retina of wild-type (wt) and R26Y^{rd1} mice either untreated (rdl NT) or transplanted with BIO-treated HSPCs (rdl BIO), all analysed at p60. Total protein lysates were normalized with an anti-β-actin antibody. ONL: outer nuclear layer; INL: inner nuclear layer; GCL: ganglion cell layer.

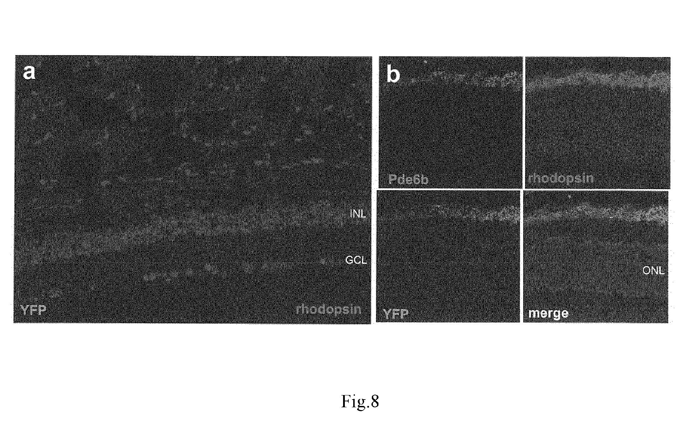

**Figure 8****.** YFP positive hybrids express *PDE6B*. (a) Representative immunofluorescence staining of rhodopsin (red) in retinal section from R26Y*^{rd1}* mice 2 months after transplantation at p10 of untreated HSPCs^{CRE} cells. Neither YFP hybrids (green) nor rhodopsin (red) positive photoreceptors were detected. Nuclei were counterstained with DAPI. (b) Representative retinal sections of R26Y^{rd1} mice transplanted at p 10 with BIO-treated HSPCs^{CRE} and analysed at p60. YFP positive hybrids (green) are positive to both rhodopsin (red) and Pde6b (magenta). Nuclei in merged images were counterstained with DAPI (blue). ONL: outer nuclear layer; INL: inner nuclear layer; GCL: ganglion cell layer.

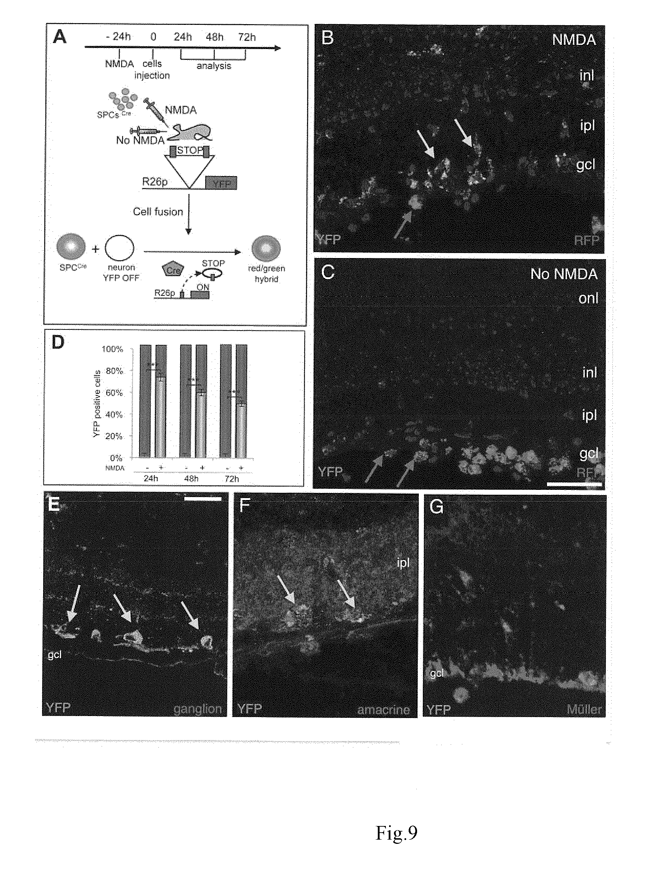

**Figure 9****.** Damage-dependent cell fusion *in-vivo.* **(A)** Schematic representation of cell fusion experimental plan. *In-vivo* cell fusion between red -labelled SPCs^{Cre} with retinal neurons of LoxP-STOP-LoxP-YFP mice (R26Y) leads to excision of a floxed stop codon in the retinal neurons, and in turn, to expression of YFP. The resulting hybrids express YFP and are also labelled in red. **(B, C)** Confocal photomicrographs of R26Y NMDA-damaged (B) or healthy retinas (C) of mice transplanted with HSPCs^{RFP/Cre}. The mice were sacrificed 24 h after tissue damage. Double-positive RFP (red) and YFP (green) hybrids derived from cell fusion are detected in the presence of NMDA damage (B, NMDA), but not in the non-damaged eye (C, No NMDA). Nuclei were counterstained with DAPI (blue). **onl:** outer nuclear layer; **inl:** inner nuclear layer; **gcl:** ganglion cell layer. Scale bar: 50 µm. **(D)** Quantification of hybrids formed 24 h after cell transplantation, as percentages of YFP-positive cells on the total red HSPCs^{Cre/RFP} localised in the optical fields. Sections of NMDA-damaged and non-damaged (No NMDA) eyes were analysed. Data are means ±s.e.m.; n = 90 (three different retinal fields of 10 different retinal serial sections, for each eye. Three different eyes were analysed). ***P <0.001. **(E-G):** Immunohistochemical analysis of the retinal fusion cell partners. YFP hybrids also positive for ganglion (E, Thy1.1, red), amacrine (F, sintaxin, red) or negative for Müller (G, GS, red) cell markers are detected 12 h after transplantation of HSPCs^{Cre} in NMDA-damaged eyes. Yellow arrows indicate cells positive to both YFP and marker staining. Scale bar: 10 µm.

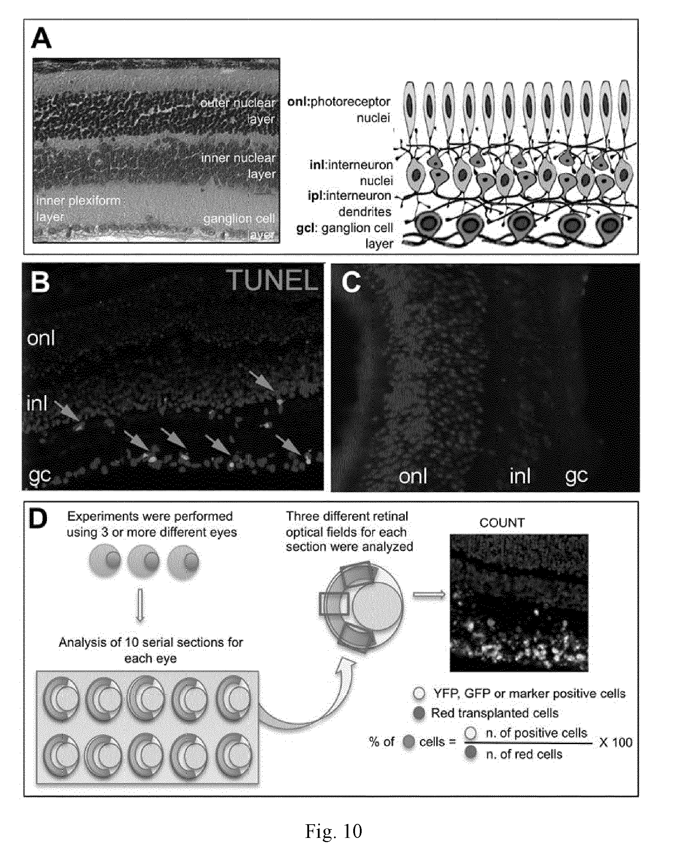

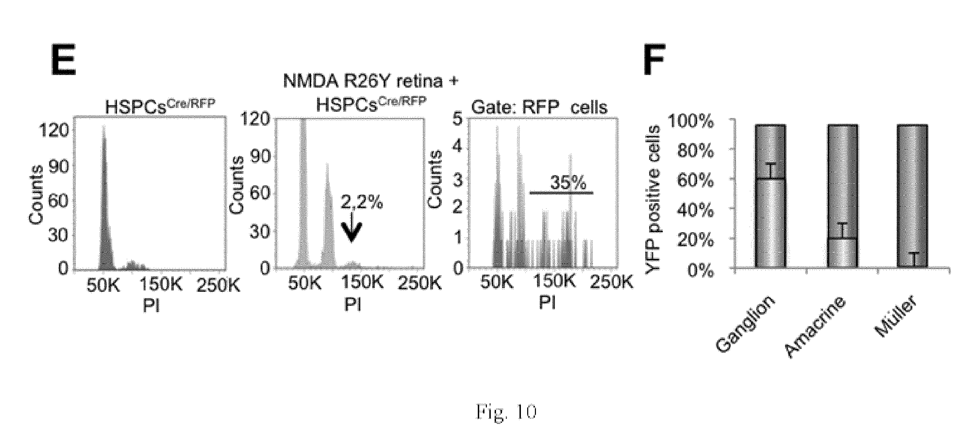

**Figure 10****.** Analysis of cell fusion events. **(A)** H&E staining (left) and schematic representation of the retinal tissue. **(B)** TUNEL staining (green) on sections of R26Y mice eyes sacrificed 48h after NMDA injection. **(C)** NMDA treatment in R26Y mice does not activate YFP expression (green) in retinal neurons. **(D)** Cell transplantation was performed at least in 3 different eyes for each experiment. Then, a total of ten serial sections from each of the eyes were examined in three different regions for each section. The number of immunoreactive marker positive, of YFP-positive or GFP- positive cells within three areas (40X optical fields) of the retina was counted in individual sections. The ratio between the latter numbers and the total number of red-labelled (DiD) cells or RFP positive cells in the same fields resulted in the percentage of positive cells. The 40X fields (red rectangles) were chosen in areas including the gcl and the inl of the retinal tissue. **(E)** Flow cytometry analysis of tetraploid cells with a 4C content of DNA was performed on total cells isolated from NMDA-damaged R26Y retinas transplanted with BIO-HSPC^{Cre}. The presence of tetraploid cells in a G2/M phase of the cell cycle was detected when gating on the RFP positive cells (hybrids) (right graph) while were not in control unfused RFP HSPCs^{Cre} (left graph). **(F)** Statistical analysis of the retinal fusion partners. Numbers represent the percentage of YFP hybrids also positive either for a ganglion, amacrine or Müller retinal cell markers detected 12 h after transplantation of HSPCs^{Cre} in NMDA-damaged R26Y eyes.

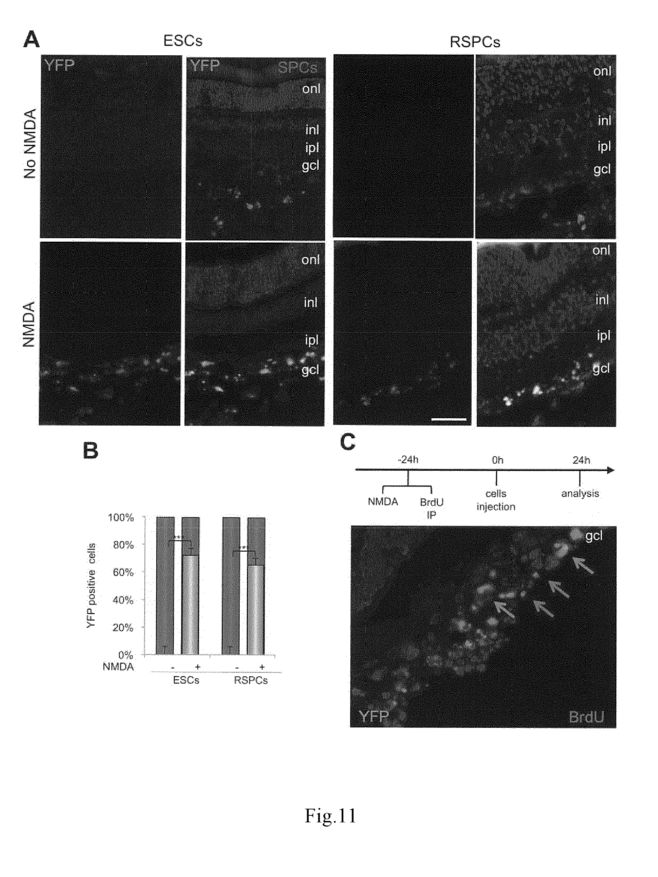

**Figure 11****.** Analysis of ESC and RSPC fusion events. **(A)** Representative samples of DiD-ESCs^{Cre} and DiD-RSPCs^{Cre} injected either into mice eyes pre-treated for 24 h with NMDA to induce cellular damage, or in healthy eyes (No NMDA). In R26Y eyes 24 h after cells injection (DiD cells, red), YFP expression (YFP, green) is detected in the NMDA-damaged eyes (NMDA), but not in the non-damaged eye (No NMDA). Nuclei were counterstained with DAPI (blue). Scale bar: 20 µm. **(B)** Quantification of YFP-positive cells as percentage relative to the total number of transplanted DiD- ESCs^{Cre} and DiD-RSPCs^{Cre} localized in the optical fields. Sections of NMDA-damaged and non-damaged eyes were analysed in mice sacrificed 24h after transplantation. Data are means ±s.e.m.; n=30 (three different areas of 10 different retinal serial sections for each eye). P value <0.001 (***). **(C)** NMDA (intravitreal) and BrdU (intraperitoneal) were injected in R26Y mice; one day later, unlabelled ESCs were injected (intravitreal) and finally BrdU staining was performed on eye sections of mice sacrificed after further 24 h. Total BrdU positive cells (red arrows) were counted in the gcl in a 40X field. YFP positive hybrids were never positive to BrdU staining (green arrows). Data are means ±s.e.m.; n=30.

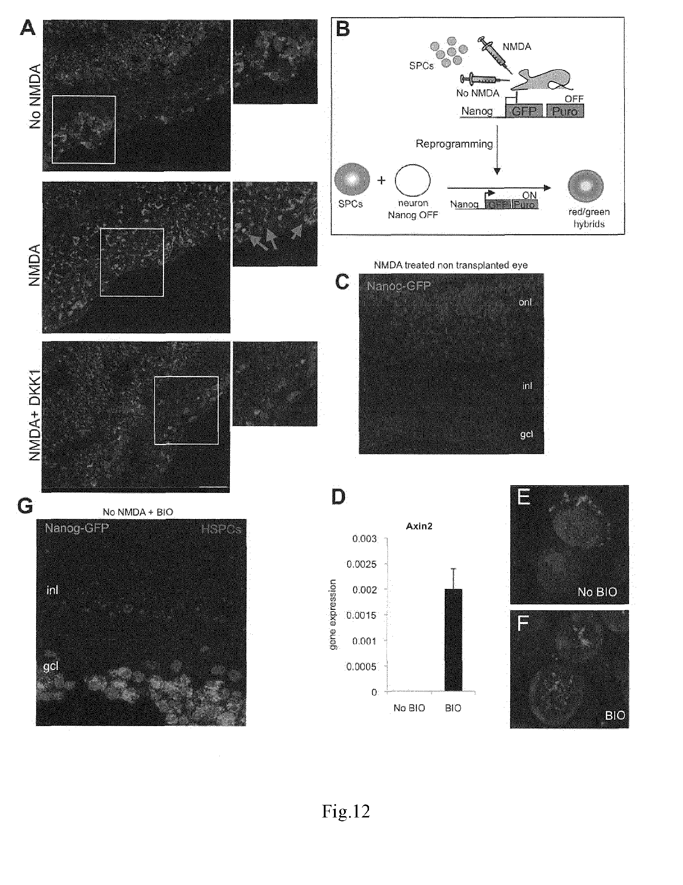

**Figure 12****.** Analysis of reprogramming of retinal neurons after fusion. **(A)** Immunofluorescence staining using an anti β-catenin antibody (red) was performed on sections from eyes treated either with NMDA, with both NMDA and DKK1 or untreated as control. The expression and nuclear accumulation of β-catenin in retinal cells detected in NMDA-damaged eyes (red arrows) is reduced after treatment with DKK1. Scale bar: 20µm. **(B)** Schematic representation of *in-vivo* reprogramming experimental plan. Red-labelled SPCs either non-treated (control), or treated for 24 h with BIO were injected in NMDA-damaged or undamaged eyes of Nanog-GFP-Puro recipient mice. The expression of GFP in reprogrammed hybrids was analysed one day after injection. **(C)** NMDA treatment does not activate GFP expression (green) in Nanog-GFP retinal neurons. **(D-F)** BIO treatment of HSPCs activates β-catenin signalling as shown by RT-PCR of the target gene Axin2 (D) or by nuclear translocation of β-catenin in untreated (E) or BIO-treated (F) cells. **(G)** Transplantation of BIO-treated HSPCs^{RFP} (red) in healthy Nanog-GFP eyes does not induce reactivation of the Nanog-GFP transgene (green). Nuclei were counterstained with DAPI.

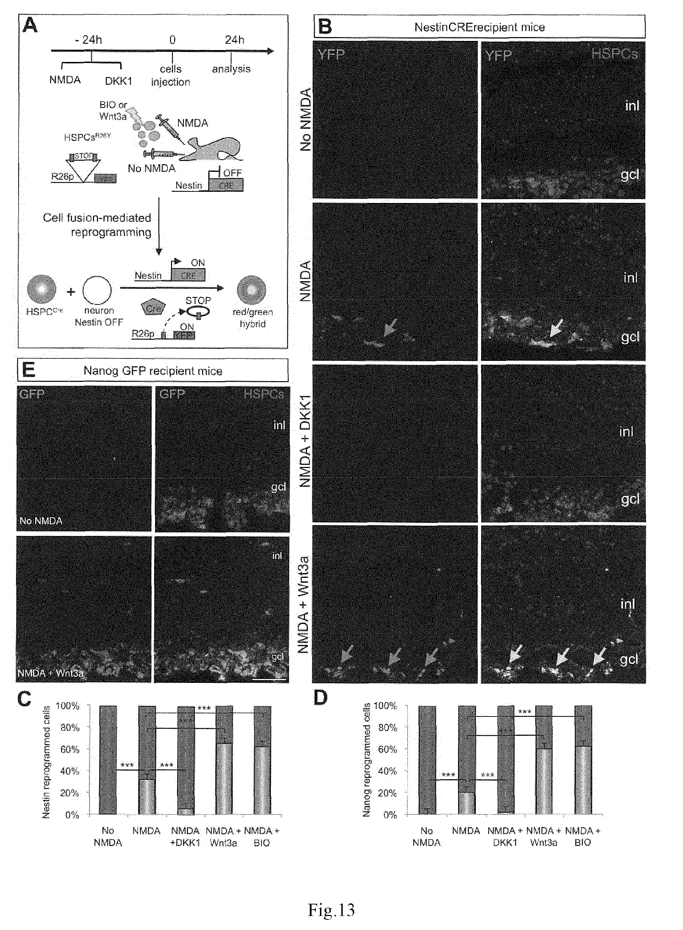

**Figure 13****.** Activation of the Wnt signalling pathway enhances neuron reprogramming after cellfusion *in-vivo.* **(A)** Schematic representation of *in-vivo* reprogramming experimental plan. Nestin-CRE mice received intravitreal injection of both NMDA and DKK1, NMDA alone, or PBS as control, one day before HSPCs^{R26Y} injection. Before transplantation, HSPCs^{R26Y} were pre-treated or not with Wnt3a or BIO and labelled with DiD red dye. Samples were analysed 24 h after cell transplantation. Only as a consequence of cell fusion and reprogramming can the Cre, re-expressed in adult mice due to the activation of the Nestin promoter, induce expression of YFP in hybrids that retain the red membranes. **(B)** Only in the presence of NMDA without DKK1 do transplanted red HSPCs^{R26Y} start to express YFP (green arrows). Yellow arrows indicate double-positive red and green cells. Wnt3a pre-treatment of red HSPCs^{R26Y} before transplantation increases the amounts of double-positive red/green hybrids. Scale bar: 50 µm **(C-D)** Statistical analysis of the numbers of double red/green (DiD/YFP)-positive hybrids detected in Nestin-CRE (C) or Nanog GFP (D) retinas treated with NMDA, NMDA+DKK1, or untreated (No NMDA), 24 h after transplantation of untreated HSPCs or of Wnt3a- or BIO-treated HSPCs. Percentages were calculated as the number of YFP-positive cells with respect to the total number of red HSPCs detected in the optical fields. Data are means ±s.e.m.; n=90. ***P <0.001. (E) Confocal photomicrographs 24h after transplantation of undamaged (No NMDA) Nanog-GFP retinas and of NMDA Nanog-GFP retinas transplanted with HSPCs pretreated with Wnt3a (NMDA + Wnt3a).

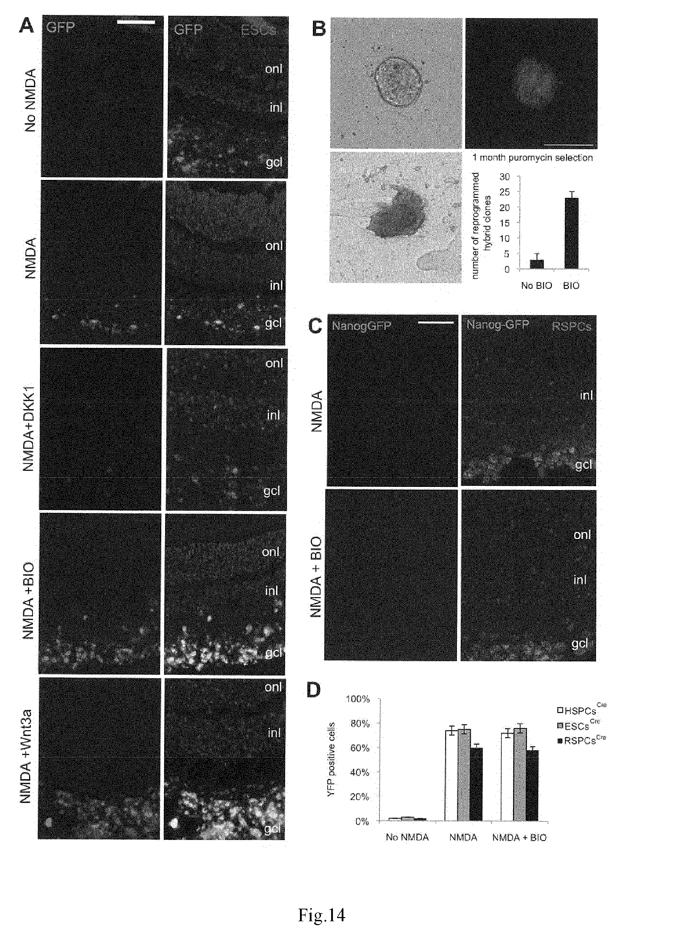

**Figure 14****.** Activation of the Wnt signalling pathway enhances neuron reprogramming after cell fusion *in vivo.* **(A)** Representative samples where DiD-ESCs were injected 24h after PBS injection (No NMDA) or NMDA injection in Nanog-GFP-puro mice. Twenty-four hours after ESC injection, Nanog-GFP expression (green) is detected in ESC-neuron hybrids (red and green) in NMDA-damaged (NMDA) but not in non-treated eyes (No NMDA). Pre-treatment with DKK1 (NMDA+DKK1) reduces the number of GFP-positive hybrids. BIO and Wnt3a pre-treatment of ESCs augmented the number of GFP-positive reprogrammed neurons (red/green) with respect to the non-treated ESCs (No BIO). Nuclei were counterstained with DAPI (blue). Scale bar: 20 µm. **(B)** Hybrids isolated from NMDA-damaged Nanog-GFP eyes transplanted with BIO-treated (BIO) or untreated (No BIO) ESCs where cultured *in vitro* under puromycin selection. A mean of 23 GFP-positive clones where detected after one month of cell culturing. Clones are also positive to the alkaline phosphatase staining. **(C)** Transplanted RSPCs (red) do not reprogram NMDA-damaged retinal neurons in presence or not of BIO treatment. Nuclei were counterstained with DAPI (blue). **(D)** Statistical analysis of the percentage of YFP- hybrids after injection of either untreated or BIO-treated HSPCs^{Cre} (white bars), ESCs^{Cre} (gray bars) or RSPCs^{Cre} (black bars), in R26Y eyes pre-treated (NMDA) or not (No NMDA) with NMDA.

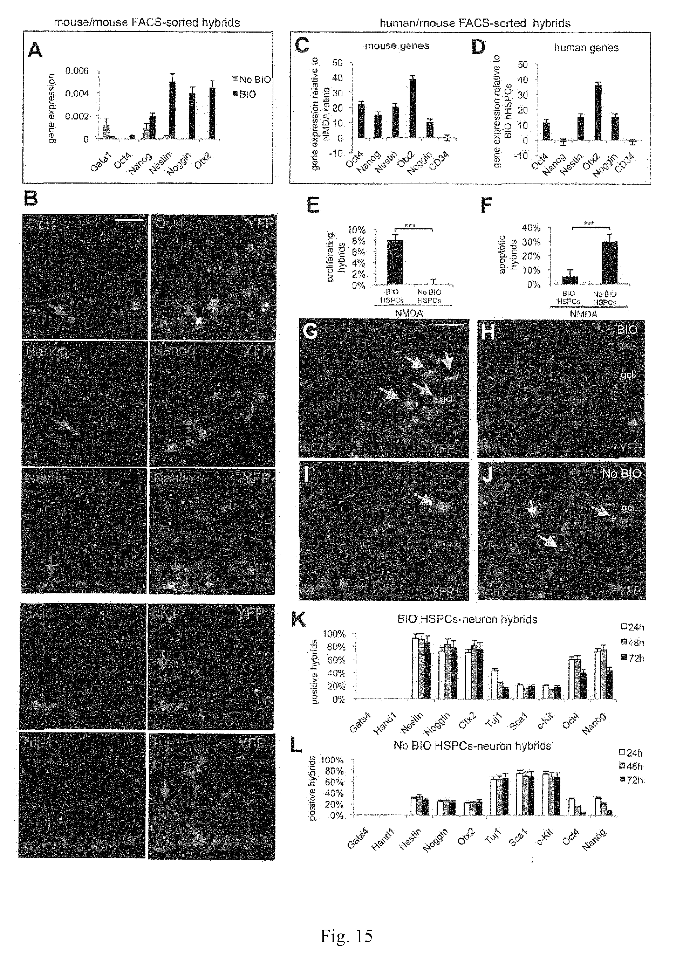

**Figure 15****.** Characterisation of the reprogrammed hybrids. **(A)** RT-PCR analysis of the expression of different genes in RFP positive hybrids sorted by FACS 24 h after transplantation of BIO (black bars) or non-BIO-treated (grey bars) HSPCs^{Cre/RFP} in R26Y NMDA-damaged eyes. **(B)** Confocal photomicrographs of NMDA-damaged R26Y retinas transplanted with BIO-treated HSPCs^{Cre} and stained 24 h later with anti-Oct4, anti-Nanog, anti-Nestin, anti cKit or anti Tuj-1 antibodies. YFP positive hybrids (green) were also positive to Oct4, Nanog and Nestin (red, arrows) expression, however they were not positive to c-Kit or Tuj-1 (green, arrows). Scale bar: 50 µm. **(C-D)** Species-specific gene expression was evaluated by RT-PCR using mouse (C) or human (D) specific oligos in hybrids FACS-sorted 24h after transplantation of BIO-treated and DiD labelled human CD34+ HSPCs in NMDA-damaged eyes of Nanog-GFP mice. **(E-J)** NMDA-damaged R26Y eyes were intravitreally injected with BIO treated (BIO) or untreated (No BIO) HSPCs^{Cre} and analyzed 24h later. To evaluate proliferation (E-G and I) and cell death (F, H and J) of YFP positive hybrids (green), sections were stained either with anti-Ki67 (G and I, red) or anti-Annexin V (H and J) antibodies. The amount of positive hybrids was evaluated as the percentage of Ki67 (E) or Annexin V (F) positive cells relative to the total number of YFP hybrids. Data are means +s.e.m.; n=30. P value <0.001 (***). Yellow arrows in G and J indicated Ki67 positive or Annexin V positive hybrids respectively. Scale bar: 50 µm. **(K-L)** The expression of markers for ESCs (Oct4, Nanog), mesoderm (Gata4), endoderm (Hand1), neuroectoderm (Nestin, Noggin and Otx2), HSPCs (c-Kit and Sca1) or terminally differentiated neurons (Tuj-1) were evaluated in YFP hybrids formed after BIO-treated (K) or non-treated (L) HSPCs^{Cre} injected into NMDA-damaged eyes of R26Y mice and sacrificed 24 (white bars), 48 (grey bars) and 72 h (black bars) after cell transplantation. Data are means +s.e.m.; n=30.

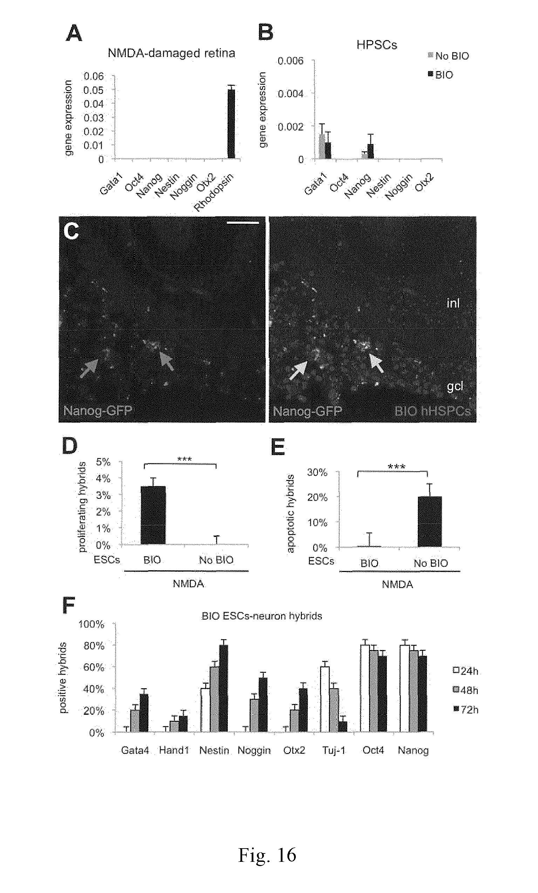

**Figure 16****.** Proliferation and gene expression in the hybrids. **(A-B)** RT-PCR analysis of untransplanted NMDA-damaged R26Y retinas (A) or of untreated (No BIO, grey bars) or BIO-treated (BIO, black bars) HSPCs cells. **(C)** Confocal photomicrographs of NMDA-damaged Nanog-GFP retinas transplanted with DiD-labelled and BIO-treated human CD34+ HSPCs (red). YFP positive hybrids (green/red cells, yellow arrows) were detected. **(D-E)** Ki67 (D) and Annexin V (E) staining were performed on YFP-positive reprogrammed hybrids obtained after injection of BIO-treated or untreated ESCs in NMDA-damaged R26Y eyes. Positive hybrids were evaluated as the percentage of positive cells relative to the total number of YFP hybrids. Data are means ±s.e.m.; n=30. P value <0.001 (***). **(F)** The expression of markers for mesoderm (Gata4), endoderm (Hand1), neuroectoderm (Nestin, Noggin and Otx2), terminally differentiated neurons (Tuj-1) or ESCs (Oct4, Nanog) were evaluated in YFP hybrids formed after BIO-treated HSPCs^{Cre} injection into NMDA-damaged eyes of R26Y mice sacrificed 24 (white bars), 48 (grey bars) and 72h (black bars) after cell transplantation. Data are means of n=30.

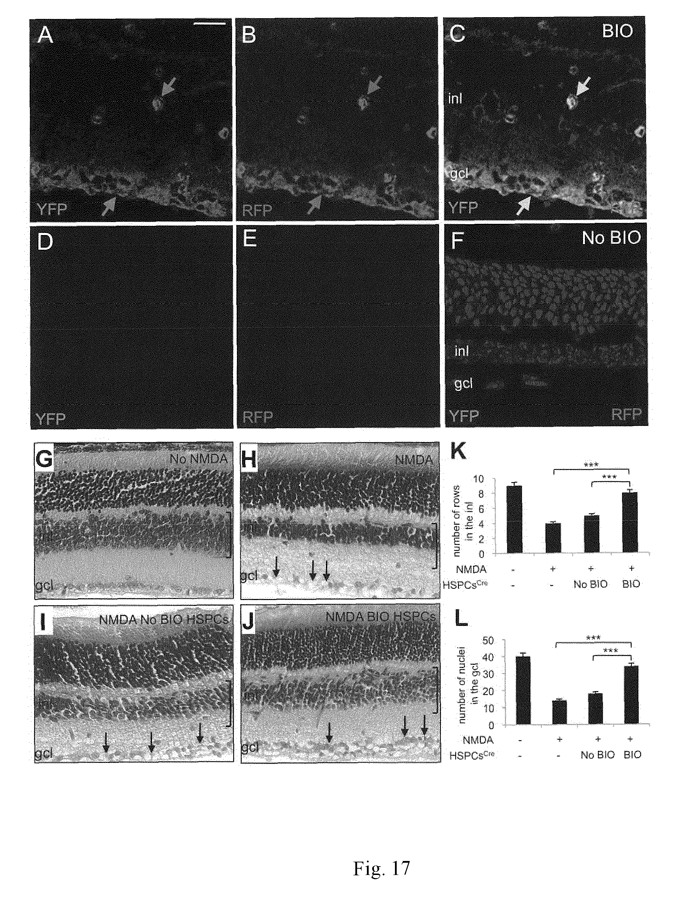

**Figure 17****.** Regeneration potential of reprogrammed hybrids obtained after cell fusion. **(A-F)** Confocal photomicrographs of YFP positive hybrids (green in A, C, D and F) obtained after transplantation of BIO treated (B-C, red) or untreated (E-F) HSPCs^{RFP/Cre} in NMDA-damaged R26Y mice analyzed 2 weeks later. YFP/RFP double positive hybrids (green and red in C and F) were detected in the inl and gcl only after transplantation of BIO-treated HSPCs. **(G-J)** H&E staining of wt (G) or NMDA-damaged R26Y retinas untreated (H) or transplanted with no-BIO (I) or BIO (J) HSPCs^{Cre}. Square brackets indicate number of nuclei in the inl. Arrows indicate presence/absence of ganglion neurons. **(K-L)** The number of interneuron (K) or ganglion (L) nuclei detected in the fields was evaluated in wt or NMDA-damaged R26Y retinas transplanted or not with no-BIO or BIO-treated HSPCs^{CRE}. Data are means ±s.e.m.; n=30. Scale bars: 20 µm.

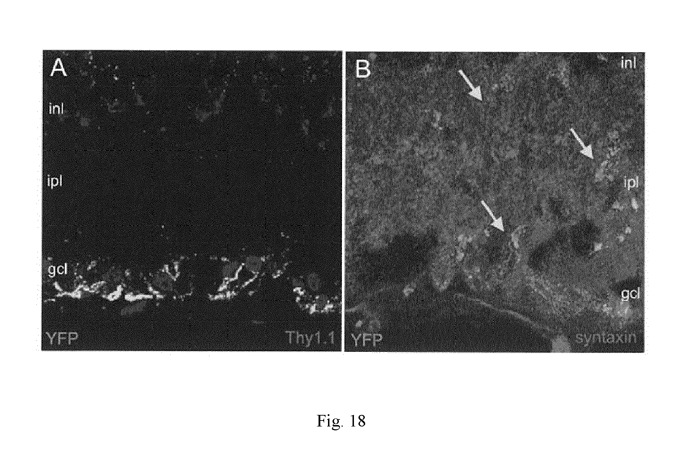

**Figure 18****.** Characterization of hybrid potential to differentiate. **(A-B)** Confocal photomicrographs of Thy1.1 (A) and syntaxin (B) staining on YFP positive hybrids (green in A and B) obtained after transplantation of BIO treated HSPCs^{Cre/RFP} in NMDA-damaged R26Y mice analyzed 2 weeks later. YFP/RFP double positive hybrids in the inl and gcl represent regenerated ganglion (A) and amacrine (B) neurons.

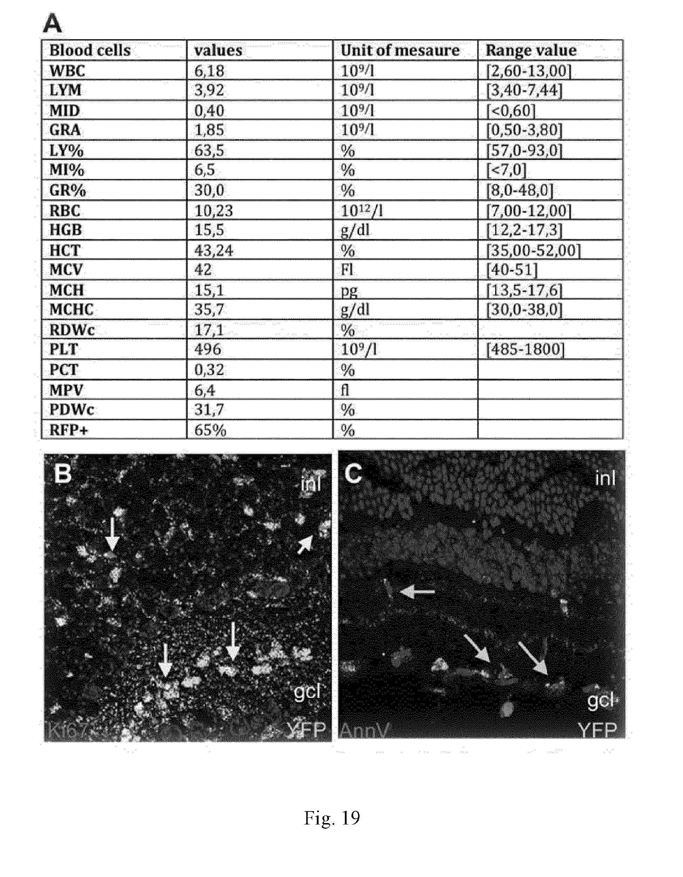

**Figure 19****.** Analysis of bone marrow replacement efficiency and analysis of hybrid proliferation and apoptosis after endogenous BM mobilization and cell fusion.**(A)** Representative haemochromocytometric analysis of mice one month after bone marrow replacement. **(B-C)** Ki67 (B) and Annexin V (C) staining were performed on YFP-positive reprogrammed hybrids obtained 24h after injection of BIO in NMDA-damaged R26Y eyes from mice that received BM^{RFP/Cre} replacement.

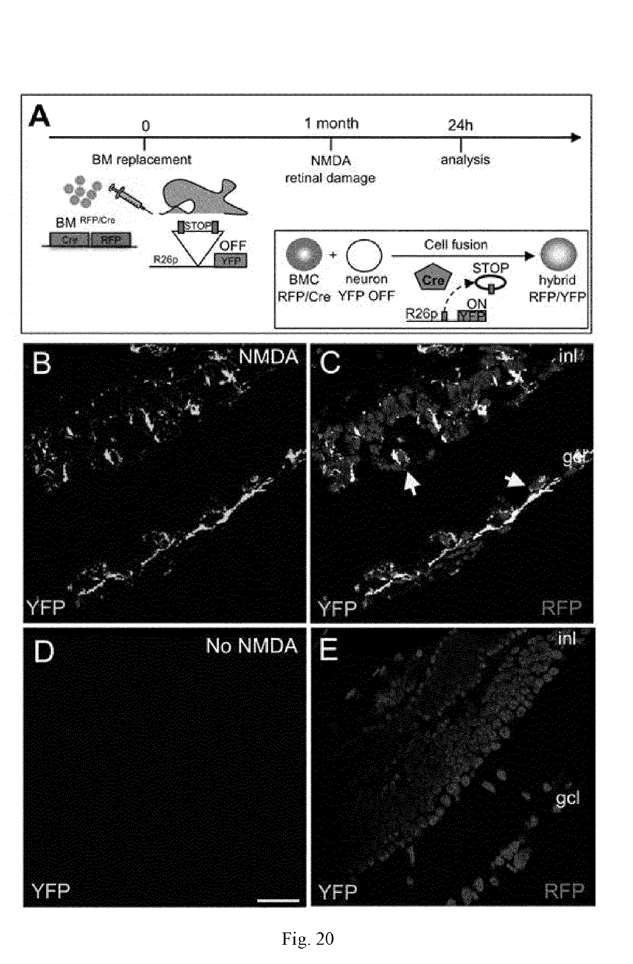

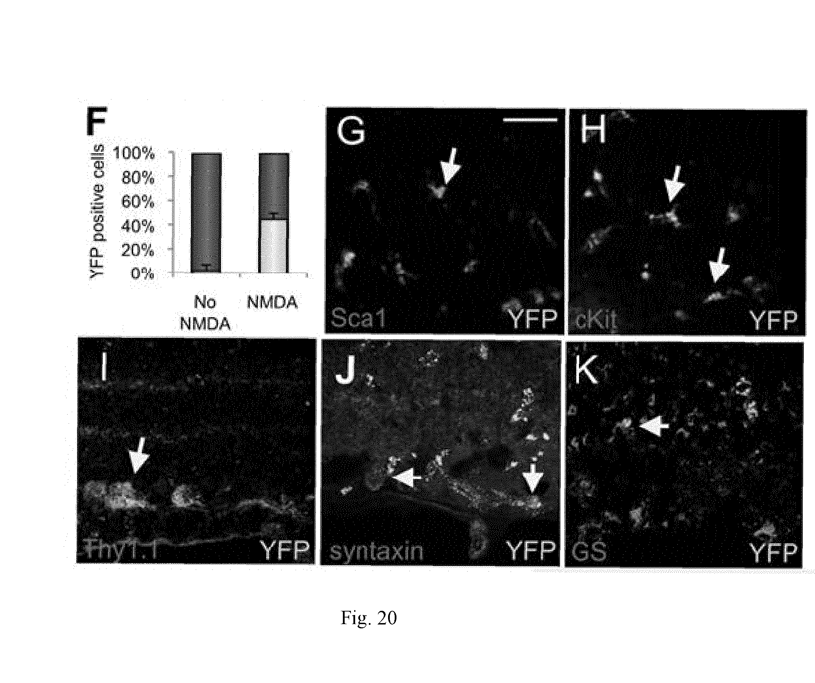

**Figure 20****.** Endogenous BM-derived cells recruited in damaged eyes can fuse with retinal neurons. **(A)** Experimental scheme. R26Y mice received BM^{RFP/Cre} transplantation via tail vein injection after sub-lethal irradiation. After BM reconstitution (1 month), right eyes received an intravitreal injection of NMDA, left eyes were not injected; the mice were analyzed 24 h later. Only in case of cell fusion of recruited-BM cells (red) and neurons, YFP/RFP double positive hybrids are detected. **(B-F)** Double positive YFP/RFP hybrids were detected in NMDA-damaged (B-C, NMDA) but not in healthy (D-E, No NMDA) eyes. (F) The percentage of YFP/RFP double positive hybrids with respect to the total number of detected RFP cells was calculated. **(G-K)** Immunohystochemical analysis of the retinal cell-fusion partners. YFP hybrids (green) are also positive for Scal (G) and c-Kit (H) HSPCs markers and for ganglion (I, Thy1.1, red), amacrine (J, syntaxin, red) and Müller (K, GS, red) retinal cell markers 24 h after NMDA damage. Yellow arrows indicate double positive cells. Scale bar: 50 µm.

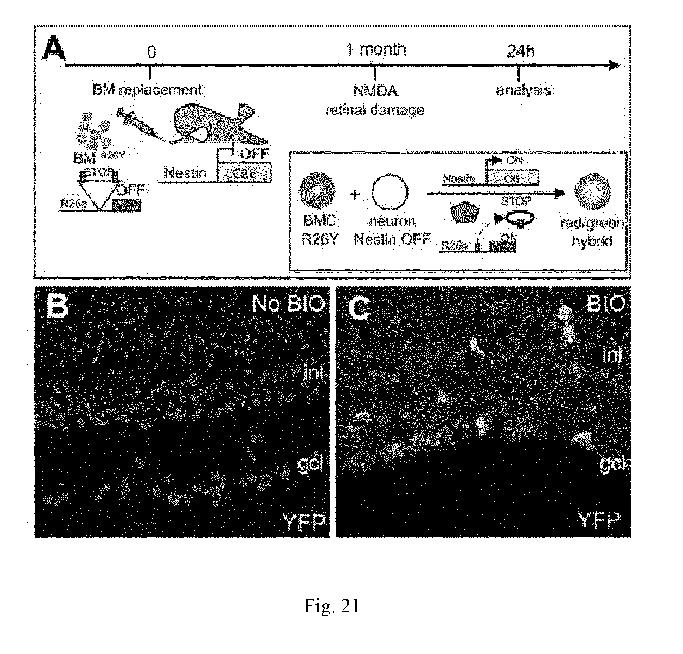

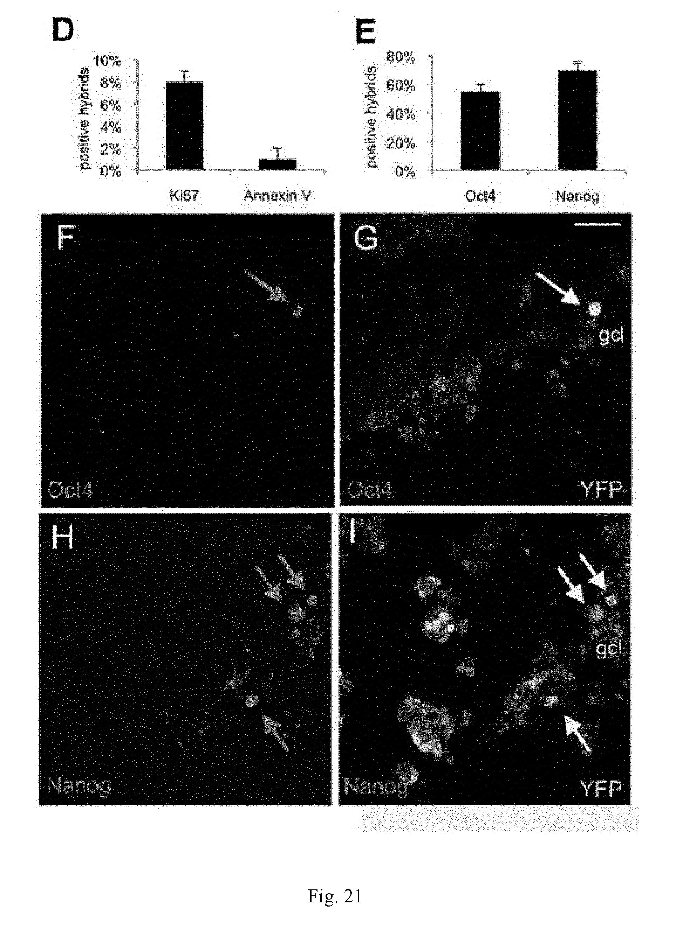

**Figure 21****.** Endogenous BM cell fusion-mediated reprogramming of retinal neurons is induced by BIO. **(A)** Experimental scheme. Nestin-Cre mice received BM^{R26Y} transplantation via tail vein inj ection after sub-lethal irradiation. After BM reconstitution (1 month), right eyes received an intravitreal injection of BIO+NMDA, while the contralateral eyes were injected with NMDA alone. Only in case of cell fusion-mediated reprogramming of hybrids between recruited-BMCs^{R26Y} and neurons, Nestin-mediated Cre expression leads to expression of the YFP. **(B-C)** Only after BIO injection (C), YFP positive reprogrammed hybrids (green) after fusion of recruited BM-cells and damaged neurons were detected. In contrast no YFP hybrids (B) were seen in NMDA-damaged eyes without BIO. **(D-E)** Percentages of proliferating (Ki67 positive, D) or dying (AnnexinV positive, E) hybrids were evaluated as the number of YFP double positive cells with the respect to the total amount of YFP cells. Data are means ±s.e.m.; n=30. **(F-I)** Confocal photomicrographs of NMDA+BIO treated retinas show expression of Oct4 (red in F-G) and Nanog (red in H-I) proteins in YFP-reprogrammed hybrids (green, see merge in G and I). Percentages of Oct4 and Nanog positive hybrids (E) were evaluated as the number of YFP double positive cells with the respect to the total amount of YFP cells. Data are means ±s.e.m.; n=30.

### DETAILED DESCRIPTION OF THE INVENTION

Retinal regeneration can be achieved by implanting pluripotent cells into the retina of a subject. These cells fuse with retinal neurons to form hybrid cells which in turn de-differentiate and finally differentiate in retinal neurons of interest, e.g., photoreceptor cells, wherein activation of Wnt/β-catenin signalling pathway in the implanted pluripotent cells or in the hybrid cells is essential to induce de-differentiation of said hybrid cells and final re-differentiation in the retinal neurons of interest. In an embodiment, activation of the Wnt/β-catenin signalling pathway is, at least partially, provided by the implanted pluripotent cells (which have been treated with a Wnt/β-catenin pathway activator, or overexpress a Wnt/β-catenin pathway activator), whereas in another embodiment, activation of the Wnt/β-catenin signalling pathway is only provided as a result of administering a Wnt/β-catenin signalling pathway activator to the subject to be treated or as a consequence of a retinal damage or injury, as occurs in, for example, retinal degeneration diseases.

### Use of pluripotent cells for treatment of retinal degeneration diseases by reprogramming, mediated by activation of the Wnt/β-catenin pathway, of retinal neurons fused to said pluripotent cells

### Treatment A

In an aspect, the invention relates to a pluripotent cell, hereinafter referred to as the "pluripotent cell of the invention", selected from the group consisting of:
(i) a pluripotent cell treated with a Wnt/β-catenin pathway activator, and
(ii) a pluripotent cell that overexpresses a Wnt/β-catenin pathway activator, for use in the treatment of a retinal degeneration disease.

Alternatively, in other words, this aspect of the invention relates to the use of a pluripotent cell of the invention in the manufacture of a pharmaceutical composition for the treatment of a retinal degeneration disease.

According to Treatment A, activation of the Wnt/β-catenin pathway is, at least partially, provided by the implanted pluripotent cells of the invention. The subject to be treated may also have activated the Wnt/β-catenin signalling pathway after retinal damage or injury.

As used herein, a "pluripotent cell" refers in general to a primordial cell that can differentiate into a sub-group of specialized types of cells, for example, a stem cell that has the potential to differentiate into any of the three germ layers: endoderm (interior stomach lining, gastrointestinal tract, the lungs), mesoderm (muscle, bone, blood, urogenital), or ectoderm (epidermal tissues and nervous system). Pluripotent stem cells can give rise to any fetal or adult cell type. However, alone they cannot develop into a fetal or adult animal because they lack the potential to contribute to extraembryonic tissue, such as the placenta. Illustrative, non-limitative examples of pluripotent cells include adipose-derived stem cells (ASCs), amniotic stem cells, bone marrow-derived stem cells (BMSCs), cord blood-derived stem cells (CBSCs), embryonic stem cells (ESCs), fetal stem cells (FSCs), amniotic stem cells, endothelial stem cells, epidermal stem cells, haematopoietic stem and progenitor cells (HSPCs), mesenchymal stem cells (MSCs), neural stem cells (NSCx), retinal stem and progenitor cells (RSPCs), etc. In a further embodiment, the pluripotent cell is an induced pluripotent stem cell, commonly abbreviated as iPS cell or iPSC, which is a type of pluripotent stem cell artificially derived from a non-pluripotent cell, typically an adult somatic cell, by inducing a "forced" expression of specific genes. iPSCs are similar to natural pluripotent stem cells in many respects, such as the expression of certain stem cell genes and proteins, chromatin methylation patterns, doubling time, embryoid body formation, teratoma formation, viable chimera formation, and potency and differentiability. Methods to obtain pluripotent cells are commonly known by the person skilled in the art, and have been described, for example, by Battey *et al.* (Chapter 8: Alternate Methods for Preparing Pluripotent Stem Cells. *Regenerative Medicine.* Department of Health and Human Services. August 2006. </info/scireport/2006report.htm>). If iPSCs are to be used, then they should be first committed *in vitro* to differentiate in a cell type of a specific lineage, e.g., a retinal neuron, etc., and then implanted in the subject.

A "progenitor cell" refers to a cell that is derived from a stem cell by differentiation and is capable of further differentiation to more mature cell types. Progenitor cells typically have more restricted proliferation capacity as compared to stem cells.

Preferably the pluripotent cell is an adult stem cell, i.e., an undifferentiated cell (called also somatic stem cell) that is present in adult or fetal or new born tissues and is able to differentiate into specialized cells of the tissue from which it originated. Such cell may be obtained by different methods known in the art, also not implying destruction of human embryos. Even more preferably the pluripotent cell is a precursor cell, i.e., a cell that detains a precocious state of differentiation and is already committed to differentiate into cell types of a specific lineage. The pluripotent cell is a mammalian cell, preferably a human cell.

In a particular embodiment, the pluripotent cell is a HSPC, such as a human CD34⁺ HSPC, an ESC, or a RSPC. HSPCs are multipotent stem cells found in bone marrow (BM) of adults that give rise to all the blood cell types from the myeloid and lymphoid lineages; in a particular embodiment said HSPCs are Lineage-negative (Lin⁻) HSPCs. ESCs are undifferentiated cells that are present in embryos and are able to differentiate into any type of specialized cell. Such ESCs, though present in embryos, may be obtained by different methods known in the art. RSPCs are undifferentiated cells located in the periphery of the retina, for example, in the ciliary marginal zone (CMZ), of adults.

For use within the teachings of the present invention, the pluripotent cell may be from the same subject, i.e., autologous, in order to minimize the risk of eventual rejections or undesired side reactions; nevertheless, the invention also contemplates the use of allogeneic pluripotent cells, i.e., cells from other subject of the same species as that of the recipient subject in which case the use of systemic or local immunosuppressive agents may be recommended, although the retina has low immune response, and, therefore, compatible pluripotent cells form a different human subject could be used.

The expression "Wnt/β-catenin pathway" refers to a network of proteins that play a variety of important roles in embryonic development, cell differentiation, and cell polarity generation. Unless otherwise indicated, it refers to the canonical Wnt pathway and includes a series of events that occur when Wnt proteins bind to cell-surface receptors of the Frizzled family, causing the receptors to activate Dishevelled family proteins and ultimately resulting in a change in the amount of β-catenin that reaches the nucleus. Dishevelled (DSH) is a key component of a membrane-associated Wnt receptor complex, which, when activated by Wnt binding, inhibits a second complex of proteins that includes axin, glycogen synthase kinase 3 (GSK-3), and the protein adenomatous polyposis coli (APC). The axin/GSK-3/APC complex normally promotes the proteolytic degradation of the β-catenin intracellular signaling molecule. After this β-catenin destruction complex is inhibited, a pool of cytoplasmic β-catenin stabilizes, and some β-catenin, is able to enter the nucleus and interact with TCF/LEF family transcription factors to promote specific gene expression. Several protein kinases and protein phosphatases have been associated with the ability of the cell surface Wnt-activated Wnt receptor complex to bind axin and disassemble the axin/GSK3 complex. Phosphorylation of the cytoplasmic domain of LRP by CK1 and GSK3 can regulate axin binding to LRP. The protein kinase activity of GSK3 appears to be important for both the formation of the membrane-associated Wnt/FRZ/LRP/DSH/Axin complex and the function of the Axin/APC/GSK3/β**-**catenin complex. Phosphorylation of β-catenin by GSK3 leads to the destruction of β-catenin.

A "Wnt/β-catenin pathway activator", as used herein, refers to a molecule capable of activating the Wnt/β-catenin pathway. Wnt/β-catenin pathway activators can act on membrane receptors of Wnt signaling proteins and on the proteins that comprise the signaling cascade. Illustrative, non-limiting examples of Wnt/β-catenin pathway activators include both Wnt/β-catenin pathway activators and glycogen synthase kinase-3 (GSK-3) inhibitors. Depending on the nature of the compound, Wnt/β-catenin pathway activators may be a peptide or a non-peptide drug, for example:
- peptides or proteins, for example, Wnt protein isoforms such as Wnt1, Wnt2, Wnt2b/13, Wnt3, Wnt3a, Wnt4, Wnt5a, Wnt5b, Wnt6, Wnt7a, Wnt8a, Wnt8b, Wnt9a, Wnt9b, Wnt10a, Wnt10b, Wnt11 or Wnt16; β-catenin; etc.; or functional variants thereof, i.e., peptides or proteins that have an amino acid sequence that is at least 70% identical to the amino acid sequences of the previously mentioned peptides or proteins and that maintain the ability to activate the Wnt/β-catenin pathway; or
- chemical compounds other than peptides or proteins (i.e., non-peptide drugs), for example, lithium salts (e.g., lithium chloride), 6-bromoindirubin-3'-oxime (BIO), 6-bromoindirubin-3'-acetoxime (BIO-Acetoxime), 6-{2-[4-(2,4-dichlorophenyl)-5-(4-methyl-1H-imidazol-2-yl)-pyrimidin-2-ylamino]-ethyl-amino}-nicotino-nitrile (CHIR99021), *N*-[(4-methoxyphenyl)methyl]-*N*'-(5-nitro-2-thiazolyl)urea (AR-A014418), 3-(2,4-dichlorophenyl)-4-(1-methyl-1*H*-indol-3-yl)-1*H*-pyrrole-2,5-dione (SB-216763), 5-benzylamino-3-oxo-2,3-dihydro-1,2,4-thiadiazole (TDZD-20), 3-[(3-Chloro-4-hydroxyphenyl)amino]-4-(2-nitrophenyl)-1*H*-pyrrole-2,5-dione (SB415286), etc., or functional analogs or derivatives thereof, i.e., compounds which contain functional groups which render the compound of interest when administered to a subject; the mentioned compounds [lithium salts (e.g., lithium chloride), BIO and CHIR99021] are known GSK-3 inhibitors.

Further examples of GSK-3 inhibitors are known to those skilled in the art. Examples are described in, for example, WO 99/65897 and WO 03/074072 and references cited therein. For example, various GSK-3 inhibitor compounds are disclosed in US 2005/0054663, US 2002/0156087, WO 02/20495 and WO 99/65897 (pyrimidine and pyridine based compounds); US 2003/0008866, US 2001/0044436 and WO01/44246 (bicyclic based compounds); US 2001/0034051 (pyrazine based compounds); and WO 98/36528 (purine based compounds). Further GSK-3 inhibitor compounds include those disclosed in WO 02/22598 (quinolinone based compounds), US 2004/0077707 (pyrrole based compounds); US 2004/0138273 (carbocyclic compounds); US 2005/0004152 (thiazole compounds); and US 2004/0034037 (heteroaryl compounds). Further GSK-3 inhibitor compounds include macrocyclic maleimide selective GSK-3 β inhibitors developed by Johnson & Johnson and described in, for example, Kuo et al. (2003) J Med Chem 46(19):4021-31, a particular example being 10,11,13,14,16,17,19,20,22,23-Decahydro-9,4:24,29-dimetho-1H -dipyrido (2,3-n:3',2'-t) pyrrolo (3,4-q)-(1,4,7,10,13,22) tetraoxadiazacyclotetracosine-1,3 (2H)-dione. Further, substituted aminopyrimidine derivatives CHIR 98014 (6-pyridinediamine, N6-[2-[[4-(2,4-dichlorophenyl)-5-(1H-imidazol-1-yl)-2-pyrimidinyl]amino]ethyl]-3-nitro-) and CHIR 99021 (6-{2-[4-(2,4-dichloro-phenyl)-5-(4-methyl-1H-imidazol-2-yl)-pyrimidin-2-ylamino]-ethylamino}-nicotinonitrile) inhibit human GSK-3 potently. Also, a number of other GSK-3 inhibitors which may be useful in the present invention are commercially available from Calbiochem®, for example: 5-methyl-1H-pyrazol-3-yl)-(2-phenylquinazolin-4-yl)amine, 4-benzyl-2-methyl-1,2,4-thiadiazolidine-3,5-dione (TDZD8), 2-thio(3-iodobenzyl)-5-(1-pyridyl)-[1,3,4]-oxadiazole, 3-(1-(3-hydroxypropyl)-1H-pyrrolo[2,3-b]pyridin-3-yl]-4-pyrazin-2-yl-pyrrole-2,5-dione, etc. Included within the scope of the invention are the functional analogs or derivatives of the above mentioned compounds.

Examples of Wnt protein isoforms include the following or orthologues thereof (Swiss-prot references):
*Homo sapiens:* Wnt1: P04628; Wnt2: P09544; Wnt2b/13: Q93097; Wnt3: P56703; Wnt3a: P56704; Wnt4: P56705; Wnt5a: P41221; Wnt5b: Q9H1J7; Wnt6: Q9Y6F9; Wnt8a: Q9H1J5; Wnt9a: 014904; Wnt9b: 014905; Wnt10a: Q9GZT5; Wnt10b: 000744; Wnt11: 096014; Wnt16: Q9UBV4.
*Mus musculus:* Wnt1: P04426; Wnt2: P21552.1; Wnt2b/13: 070283.2; Wnt3: P17553; Wnt3a: P27467; Wnt4: P22724; Wnt5a: P22725; Wnt5b: P22726;
Wnt6: P22727.1; Wnt8a: Q64527; Wnt9a: Q8R5M2; Wnt9b: 035468.2; Wnt10a: P70701; Wnt10b: P48614; Wnt11: P48615; Wnt16: Q9QYS1.1.

Examples of β-catenin include the following or orthologues thereof (Swiss-prot references):
*Homo sapiens:* P35222.
*Mus musculus:* Q02248.

In a particular embodiment, the Wnt activator is Wnt3a, β-catenin, BIO or CHIR99021.

In a particular embodiment, the pluripotent cell of the invention is a pluripotent cell treated with a Wnt/β-catenin pathway activator. According to this embodiment, a pluripotent cell is contacted, e.g., cultured or incubated, with a Wnt/β-catenin pathway activator. The amount of said Wnt/β-catenin pathway activator may vary within a range; nevertheless, preferably, the Wnt/β-catenin pathway activator will be added in a suitable amount, i.e., in an amount which allows to obtain a specific amount of β-catenin accumulated in the nucleus of the pluripotent cells. A range of 100-300 ng/ml Wnt3a or 1-3 µM BIO may be used to treat pluripotent cells in a specific culture condition (see below). Assays performed by the inventors have shown that if the Wnt/β-catenin pathway is activated too much or too little then no cell fusion-mediated reprogramming is observed. The amount of Wnt/β-catenin pathway activator which allows to obtain a specific amount of β-catenin accumulated in the cells and translocated in the nucleus of the pluripotent cells with which cell fusion-mediated reprogramming is observed can be determined by the skilled person in the art by means of an assay as that mentioned in Example 1. Briefly, said assay comprises contacting the pluripotent cell with a Wnt/β-catenin pathway activator, at different concentrations and during different periods of time before transplantation of the so treated pluripotent cells into an animal and then analyzing if cell fusion-mediated reprogramming occurs, for example, by detecting and/or determining the expression of undifferentiated cells markers, e.g., Nanog, Oct4, Nestin, Otx2, Noggin, SSEA-1, etc. In a particular embodiment, the pluripotent cell is treated with BIO as Wnt/β-catenin pathway activator, in a suitable amount of about 1-3 µM for 24 h before transplantation of the treated pluripotent cell. In another particular embodiment, the pluripotent cell is treated with Wnt3a as Wnt/β-catenin pathway activator, in a suitable amount of about 100-300 ng/µl for 24 h before transplantation of the treated pluripotent cell.

In another particular embodiment, the pluripotent cell is a pluripotent cell that overexpresses a Wnt/β-catenin pathway activator.

As used herein, a "pluripotent cell that overexpresses a Wnt/β-catenin pathway activator" is a pluripotent cell that has been genetically manipulated to overexpress a Wnt/β-catenin pathway activator, wherein said Wnt/β-catenin pathway activator is a peptide or protein. In a particular embodiment, said Wnt/β-catenin pathway activator is a Wnt protein isoform such as Wnt1, Wnt2, Wnt2b/13, Wnt3, Wnt3a, Wnt4, Wnt5a, Wnt5b, Wnt6, Wnt7a, Wnt8a, Wnt8b, Wnt9a, Wnt9b, Wnt10a, Wnt10b, Wnt11 or Wnt16. In another particular embodiment, said Wnt/β-catenin pathway activator is β-catenin. In an embodiment, the polynucleotide comprising the nucleotide sequence encoding the Wnt/β-catenin pathway activator is comprised in an expression cassette, and said polynucleotide is operatively bound to (i.e., under the control of) an expression control sequence of said polynucleotide comprising the nucleotide sequence encoding the Wnt/β-catenin pathway activator. Expression control sequences are sequences that control and regulate transcription and, where appropriate, translation of a protein, and include promoter sequences, sequences encoding transcriptional regulators, ribosome binding sequences (RBS) and/or transcription terminator sequences. In a particular embodiment, said expression control sequence is functional in eukaryotic cells, such as mammalian cells, preferably human cells, for example, the human cytomegalovirus (hCMV) promoter, the combination of the cytomegalovirus (CMV) early enhancer element and chicken beta-actin promoter (CAG), the eukaryotic translation initiation factor (eIF) promoter, etc.

Advantageously, said expression cassette further comprises a marker or gene encoding a motive or for a phenotype allowing the selection of the host cell transformed with said expression cassette. Illustrative examples of said markers that could be present in the expression cassette of the invention include antibiotic-resistant genes, toxic compound-resistant genes, fluorescent marker-expressing genes, and generally all those genes that allow selecting the genetically transformed cells. The gene construct can be inserted in a suitable vector. The choice of the vector will depend on the host cell where it will subsequently be introduced. By way of illustration, the vector in which the polynucleotide comprising the nucleotide sequence encoding the Wnt/β-catenin pathway activator is introduced can be a plasmid or a vector which, when introduced in a host cell, either becomes integrated or not in the genome of said cell. Said vector can be obtained by conventional methods known by persons skilled in the art [Sambrook and Russell, "Molecular Cloning, A Laboratory Manual", 3rd ed., Cold Spring Harbor Laboratory Press, N.Y., 2001 Vol 1-3]. In a particular embodiment, said recombinant vector is a vector that is useful for transforming animal cells, preferably mammalian cells. Said vector can be used to transform, transfect or infect pluripotent cells. Transformed, transfected or infected cells can be obtained by conventional methods known by persons skilled in the art [Sambrok and Russell, (2001), cited *supra*].

The pluripotent cells of the invention, preferably isolated pluripotent cells of the invention, may be used to initiate, or seed, cell cultures. Isolated cells may be transferred to sterile tissue culture vessels, either uncoated or coated with extracellular matrix or ligands such as laminin, collagen (native, denatured or crosslinked), gelatin, fibronectin, and other extracellular matrix proteins. The pluripotent cells of the invention may be cultured in any suitable culture medium (depending on the nature of the pluripotent cell of the invention) capable of sustaining growth of the cells such as, for example, DMEM (high or low glucose), advanced DMEM, DMEM/MCDB 201, Eagle's basal medium, Ham's F10 medium (F10), Ham's F-12 medium (F12), Iscove's modified Dulbecco's - 17 medium, DMEM/F12, RPMI 1640, etc. If necessary, the culture medium may be supplemented with one or more components including, for example, fetal bovine serum (FBS); equine serum (ES); human serum (HS); betamercaptoethanol (BME or 2-ME), preferably about 0.001% (v/v); one or more growth factors, for example, platelet-derived growth factor (PDGF), epidermal growth factor (EGF), fibroblast growth factor (FGF), vascular endothelial growth factor (VEGF), insulin-like growth factor-1 (IGF-1), leukocyte inhibitory factor (LIF), stem cell factor (SCF) and erythropoietin; cytokines as interleukin-3 (IL-3), interleukin-6 (IL-6), FMS-like tyrosine kinase 3 (Flt3); amino acids, including L-valine; and one or more antibiotic and/or antimycotic agents to control microbial contamination, such as, for example, penicillin G. streptomycin sulfate, amphotericin B, gentamicin, and nystatin, either alone or in combination. The cells may be seeded in culture vessels at a density to allow cell growth.

Methods for the selection of the most appropriate culture medium, medium preparation, and cell culture techniques are well known in the art and are described in a variety of sources, including Doyle et al., (eds.), 1995, Cell & Tissue Culture: Laboratory Procedures, John Wiley &Sons, Chichester; and Ho and Wang (eds.), 1991, Animal Cell Bioreactors, Butterworth-Heinemann, Boston.

Thus, in another aspect, the invention relates to a cell composition, hereinafter referred to as the "cell composition of the invention", wherein at least 50% of the cells of said cell composition are the pluripotent cells of the invention. In a particular embodiment, the cell composition of the invention is a composition wherein at least 60%, preferably 70%, more preferably 80%, still more preferably 90%, yet more preferably 95%, and even more preferably 100% of the cells are pluripotent cells of the invention. The cell composition of the invention further comprises a medium; said medium must be compatible with said cells, namely with the pluripotent cells of the invention present in said composition; illustrative, non-limitative examples of media which can be present in the cell composition of the invention include isotonic solutions optionally supplemented with serum; cell culture media or, alternatively, a solid, semisolid, gelatinous or viscous support medium.

As it is shown in Example 1, pluripotent cells of the invention, transplanted into the subretinal space of *rd1* mice at postnatal day 10 (p10) fuse with rods and Müller cells, thus forming hybrids which de-differentiate and finally re-differentiate in retinal neurons, for example photoreceptor cells such as rods, etc. The activation of Wnt/β-catenin signalling pathway in the transplanted pluripotent cells appears to be essential to induce de-differentiation of newly formed hybrids that finally re-differentiate in newborn retinal neurons. Further, the newborn photoreceptor cells fully regenerate the retina in the transplanted mice, with rescue of functional vision. These data demonstrate that pluripotent cell fusion-mediated regeneration is a very efficient process in mammalian retina, and that it can be triggered by activation of Wnt/β-catenin signaling pathway. Retinitis Pigmentosa (RP) is a very severe disease for which no treatment is currently available. However, retinal regeneration through transplantation of the pluripotent cells of the invention constitutes an approach for the rescue of vision in patients affected by RP or even by a variety of retinal degeneration diseases.

The pluripotent cells of the invention can be used as a cell therapy for treating a retinal degeneration disease since, once transplanted into a target location in the eye, the pluripotent cells of the invention fuse with retinal neurons thus providing hybrid cells which differentiate into one or more phenotypes. According to the invention, the treatment of the retinal degeneration disease occurs by reprogramming of retinal neurons mediated by cell fusion of said pluripotent cell with said retinal neurons. Reprogramming, in general, can be referred to the passage of a cell from the differentiated state (or differentiated cell - i.e., a cell specialized for a specific function, such as a heart, liver, etc., that cannot generate other types of cells) to an undifferentiated state (or undifferentiated stem cell - i.e., a cell not specialized for a specific function that retains the potential to give rise to specialized cells), both at level of embryonic state or progenitor state; but also reprogramming can be referred to the passage from one differentiated state to another differentiated state (for example, a fibroblast that becomes a neuron without going back to a precursor/embryonic state, or a retinal neuron that becomes another retinal neuron without going back to a precursor/embryonic state). In this description, "reprogramming" refers only to the de-differentiation of a somatic cell which is followed by differentiation of the hybrid cells previously formed as a result of the cell fusion between a pluripotent cell (e.g., HSPC, ESC, RSPC, etc.) and a somatic cell (e.g., a retinal neuron).

As used herein, the expression "cell fusion" relates to cell-cell fusion that occurs spontaneously or mediated by exogenous agents. Cell-cell fusion regulates many developmental processes as well as cell fate and cell differentiation. Somatic cells can fuse spontaneously with stem cells, and the resulting hybrid clones have a stem cell-like phenotype. The stem cell features of stem cells are dominant over the somatic cell traits and allow the reprogramming of the somatic cell nucleus. Thus, cell-cell fusion is a way to force the fate of a cell, and in the case of fusion with pluripotent cells this mechanism induces cellular reprogramming, that is, dedifferentiation of somatic cells. The inventors have shown that fusion-mediated reprogramming of a somatic cell is greatly enhanced by time-dependent activation of the Wnt/β-catenin signaling pathway. After Wnt binding to its receptors or inhibition of GSK-3, as a component of the destruction complex, β-catenin is stabilized and translocates into the nucleus, where it activates several target genes.

As used herein, the term "retinal neuron" refers to the neurons which form part of the retina. The retina is a light-sensitive tissue lining the inner surface of the eye. It is a layered structure with several layers of neurons interconnected by synapses. The only neurons that are directly sensitive to light are the photoreceptor cells. These are mainly of two types: the rods and cones. Rods function mainly in dim light and provide black-and-white vision, while cones support daytime vision and the perception of colour. A third, much rarer type of photoreceptor, the photosensitive ganglion cell, is important for reflexive responses to bright daylight. Neural signals from the rods and cones undergo processing by other neurons of the retina. The output takes the form of action potentials in retinal ganglion cells whose axons form the optic nerve. The retinal neurons further include horizontal cells, bipolar cells, amacrine cells, interplexiform cells, ganglion cells, Müller cells, and, in addition to these, there are microglial cells in the retina which act as supporting cells.

In a particular embodiment, the retinal neurons are rods and Müller cells, which fuse with the pluripotent cells of the invention, e.g., BIO-treated HSPCs (Example 1). In another particular embodiment, the retinal neurons are ganglion and amacrine cells which fuse with the transplanted HSPCs (Example 2). In another particular embodiment it is contemplated the fusion of pluripotent cells, including the pluripotent cells of the invention, e.g., HSPCs, with endogenous proliferating cells (e.g., RSPCs).

The final retinal neurons which may obtained after reprogramming of the fused retinal neurons may vary, for example, photoreceptor cells, ganglion cells, interneurons, etc. In a particular embodiment, fused retinal neurons (rods and Müller cells) are reprogrammed to mainly rods (Example 1), whereas in another particular embodiment fused retinal neurons (ganglion and amacrine cells) are reprogrammed to ganglion cells and interneurons (Example 2).

Although the inventors wish not to be bound by any theory, it is believed that the reprogrammed neuron cells may be of the same type (or different) as that of the retinal neuron fused to the pluripotent cell of the invention, e.g., a rod may be reprogrammed to a rod or to another type of retinal neuron such as, e.g., a ganglion cell, a Müller cell, an amacrine cell, etc.; a ganglion cell may be reprogrammed to a ganglion cell or to another type of retinal neuron such as, e.g., a rod, a Müller cell, an amacrine cell, etc.; a Müller cell may be reprogrammed to a Müller cell or to another type of retinal neuron such as, e.g., a rod, a ganglion cell, an amacrine cell, etc.; an amacrine cell may be reprogrammed to an amacrine cell or to another type of retinal neuron such as, e.g., a rod, a ganglion cell, a Müller cell, etc. Indeed, Example 1 shows fusion of pluripotent cells (HSPCs) with both rods and Müller cells and the differentiation of the hybrid cells only into rods.

In a particular embodiment, the treatment of said retinal degeneration disease comprises reprogramming of retinal neurons mediated by cell fusion of said pluripotent cell of the invention with said retinal neurons and differentiation of the resulting hybrid cells to photoreceptor cells, e.g., rods. In another particular embodiment, the treatment of said retinal degeneration disease comprises reprogramming of retinal neurons mediated by cell fusion of said pluripotent cell of the invention with said retinal neurons and differentiation of the resulting hybrid cells to ganglion neurons, amacrine neurons, etc.

A "retinal degeneration disease", as defined herein, is a disease associated with deterioration of the retina caused by the progressive and eventual death of the cells of the retinal tissue. The term "retinal degeneration disease" also includes indirect causes of retinal degeneration, i.e., retinal degenerative conditions derived from other primary pathologies, such as cataracts, diabetes, glaucoma, etc. In a particular embodiment, said retinal degeneration disease is selected from the group comprising retinitis pigmentosa, age-related macular degeneration, Stargardt disease, cone-rod dystrophy, congenital stationary night blindness, Leber congenital amaurosis, Best's vitelliform macular dystrophy, anterior ischemic optic neuropathy, choroideremia, cone-rod dystrophy, age-related macular degeneration, foveomacular dystrophy, Bietti crystalline corneoretinal dystrophy, Usher's syndrome, etc., as well as retinal degenerative conditions derived from other primary pathologies, such as caractacts, diabetes, glaucoma, etc. In another particular embodiment, said retinal degeneration disease is age-related macular degeneration that is presented in two forms: "dry" that results from atrophy to the retinal pigment epithelial layer below the retina, which causes vision loss through loss of photoreceptors (rods and cones) in the central part of the eye; and "wet" that causes vision loss due to abnormal blood vessel growth (choroidal neovascularization) in the choriocapillaris, through Bruch's membrane, ultimately leading to blood and protein leakage below the macula, eventually causing irreversible damage to the photoreceptors and rapid vision loss. In a more particular embodiment, said retinal degeneration disease is RP, a heterogeneous family of inherited retinal disorders characterized by progressive degeneration of the photoreceptors with subsequent degeneration of RPE, which is characterized by pigment deposits predominantly in the peripheral retina and by a relative sparing of the central retina. In most of the cases of RP, there is primary degeneration of photoreceptor rods, with secondary degeneration of cones.

In the context of the present invention, "treatment of retinal degeneration disease" means the administration of the pluripotent cells of the invention or a pharmaceutical composition comprising said pluripotent cells or a pharmaceutical composition comprising pluripotent cells other than the pluripotent cells of the invention (see Treatment B below) to prevent or treat the onset of symptoms, complications or biochemical indications of a retinal degeneration disease, to alleviate its symptoms or to stop or inhibit its development and progression such as, for example, the onset of blindness. The treatment can be a prophylactic treatment to delay the onset of the disease or to prevent the manifestation of its clinical or subclinical symptoms or a therapeutic treatment to eliminate or alleviate the symptoms after the manifestation of the disease.

Survival of transplanted cells in a living subject may be determined through the use of a variety of scanning techniques, e.g., computerized axial tomography (CAT or CT) scan, magnetic resonance imaging (MRI) or positron emission tomography (PET) scans. Alternatively, determination of transplant survival may also be done post mortem by removing the tissue and examining it visually or through a microscope. Examining restoration of the ocular function that was damaged or diseased can assess functional integration of transplanted cells into ocular tissue of a subject. For example, effectiveness in the treatment of retinal degeneration diseases may be determined by improvement of visual acuity and evaluation for abnormalities and grading of stereoscopic color fundus photographs (Age- Related Eye Disease Study Research Group, NEI5 NIH, AREDS Report No. 8, 2001, Arch. Ophthalmol. 119: 1417-1436).

For the administration to a subject, the pluripotent cells of the invention may be formulated in a pharmaceutical composition, preparation or formulation, using pharmaceutically acceptable carriers, which particulars will be discussed below under section entitled "Pharmaceutical composition".

### Treatment B

In another aspect, the invention relates to a pluripotent cell for use in the treatment of a retinal degeneration disease, by reprogramming of retinal neurons mediated by cell fusion of said pluripotent cell with said retinal neurons, said reprogramming being mediated by activation of the Wnt/β-catenin pathway.

Alternatively, in other words, this aspect of the invention relates to the use of a pluripotent cell in the manufacture of a pharmaceutical composition for the treatment of a retinal degeneration disease by reprogramming of retinal neurons mediated by cell fusion of said pluripotent cell with said retinal neurons, said reprogramming being mediated by activation of the Wnt/β-catenin pathway.

The particulars of the pluripotent cell and the retinal degeneration disease to be treated have been previously discussed in connection with above Treatment A, whose particulars are hereby incorporated.

In contrast to above Treatment A, in Treatment B it is not necessary that the pluripotent cell is treated with a Wnt/β-catenin pathway activator or that overexpresses a Wnt/β-catenin pathway activator, but what is necessary is that retinal regeneration occurs by reprogramming of retinal neurons mediated by cell fusion of said pluripotent cell with said retinal neurons, said reprogramming being mediated by activation of the Wnt/β-catenin pathway. The activation of the Wnt/β-catenin pathway may be endogenous, i.e., it can be achieved by the subject to which the pluripotent cells are to be administered as a consequence of a damage, lesion or injury in the retina (what may occur in retinal degeneration diseases) or by administration of a Wnt/β-catenin pathway activator. Several assays performed by the inventors have shown that after endogenous activation of the Wnt/β-catenin pathway reprogramming of the hybrid cells formed after damage is observed (Example 2). On the other hand, recruitment of endogenous bone marrow cells (BMCs) after damage in the eye and ectopic activation of Wnt/β-catenin pathway is sufficient to observe reprogramming of the hybrid cells (Example 2). Therefore, in a particular embodiment, the pluripotent cell for use in the treatment of a retinal degeneration disease according to Treatment B (i.e., by reprogramming of retinal neurons mediated by cell fusion of said pluripotent cell with said retinal neurons, said reprogramming being mediated by activation of the Wnt/β-catenin pathway) is a BMC recruited from the bone marrow (BM) into the eye and the eye is treated with a Wnt/β-catenin pathway activator in order to obtain regeneration of the retinal tissue.

The effects of the activation of the Wnt/β-catenin pathway, as well as illustrative, non-limitative, examples of Wnt/β-catenin pathway activators have been discussed in connection with above Treatment A, whose particulars are hereby incorporated.

Example 2 shows that upon activation of Wnt/β-catenin signalling pathway, mouse retinal neurons can be transiently reprogrammed *in vivo* back to a precursor stage after spontaneous fusion with transplanted pluripotent cells (e.g., HSPCs, ESCs or RSPCs). Newly formed hybrid cells reactivate neuronal precursor markers (e.g., HSPCs and ESCs reprogramme retinal neurons back to Nanog and Nestin expression, whereas RSPCs appear to reprogramme retinal neurons only back to Nestin expression). Further, said hybrid cells can proliferate, differentiate along a neuro-ectodermal lineage (in the case of hybrid cells formed by HSPCs and retinal neurons), and finally into terminally differentiated retinal neurons (e.g., photoreceptor cells), which can regenerate the damaged retinal tissue; alternatively, hybrid cells formed by ESCs and retinal neurons can also proliferate and differentiate, in addition to the neuroectodermal lineage, in endoderm and ectoderm lineages what may result in formation of a teratoma. Following retinal damage and induction of Wnt/β-catenin signalling pathway in the eye, cell-fusion-mediated reprogramming also occurs after endogenous mobilisation of bone marrow cells in the eyes. These data show that *in-vivo* reprogramming of terminally differentiated retinal neurons is a possible mechanism of tissue regeneration.

### Pharmaceutical compositions

The pluripotent cells of the invention (Treatment A) as well as the pluripotent cells (Treatment B) may be administered in a pharmaceutical composition, preparation, or formulation, by using pharmaceutically acceptable carriers.

Thus, in another aspect, the invention relates to a pharmaceutical composition comprising at least a pluripotent cell of the invention and a pharmaceutically acceptable carrier.

Although the particulars of the pharmaceutical composition for the administration of cells to a subject will be discussed in connection with the pharmaceutical composition of the invention, which comprises the pluripotent cells of the invention, the skilled person in the art will easily understand that said particulars are also applicable to pharmaceutical composition comprising pluripotent cells other than the pluripotent cells of the invention (i.e., those used in above Treatment B) unless otherwise indicated.

As used herein, the term "carrier" includes vehicles, media or excipients, whereby the pluripotent cells of the invention can be administered. Obviously, said carrier must be compatible with said pluripotent cells. Illustrative, non-limiting examples of suitable pharmaceutically acceptable carriers include any physiologically compatible carrier, for example, isotonic solutions (e.g., 0.9% NaCl sterile saline solution, phosphate buffered saline (PBS) solution, Ringer-lactate solution, etc.) optionally supplemented with serum, preferably with autologous serum; cell culture media (e.g., DMEM, etc.); etc.

The pharmaceutical composition of the invention may comprise auxiliary components as would be familiar to medicinal chemists or biologists, for example, an antioxidant agent suitable for ocular administration (e.g., EDTA, sodium sulfite, sodium metabisulfite, mercaptopropionyl glycine, N-acetyl cysteine, beta- mercaptoethylamine, glutathione and similar species, ascorbic acid and its salts or sulfite or sodium metabisulfite, etc.), a buffering agent to maintain the pH at a suitable pH to minimize irritation of the eye (e.g., for direct intravitreal or intraocular injection, the pharmaceutical compositions should be at pH 7.2 to 7.5, alternatively at pH 7.3-7.4), a tonicity agent suitable for administration to the eye (e..g., sodium chloride to make compositions approximately isotonic with 0.9% saline solution), a viscosity enhancing agent (e.g., hydroxyethylcellulose, hydroxypropylcellulose, methylcellulose, polyvinylpyrrolidone, etc.), etc. In some embodiments, the pharmaceutical composition of the invention may contain a preservative (e.g., benzalkonium chloride, benzethonium chloride, chlorobutanol, phenylmercuric acetate or nitrate, thimerosal, methyl or propylp8arabens, etc.). Said pharmaceutically acceptable substances which can be used in the pharmaceutical composition of the invention are generally known by the persons skilled in the art and are normally used in the preparation of cell compositions. Examples of suitable pharmaceutical carriers are described, for example, in "Remington's Pharmaceutical Sciences", of E.W. Martin.

The pluripotent cells may be administered alone (e.g., as substantially homogeneous populations) or as mixtures with other cells, for example, neurons, neural stem cells, retinal stem cells, ocular progenitor cells, retinal or corneal epithelial stem cells and/or other multipotent or pluripotent stem cells. Where pluripotent cells are administered with other cells, they may be administered simultaneously or sequentially with the other cells (either before or after the other cells). The cells of different types may be mixed with the pluripotent cells immediately or shortly prior to administration, or they may be co-cultured together for a period of time prior to administration.

The pluripotent cells may be administered with at least one pharmaceutical agent, such as, for example, growth factors, trophic factors, conditioned medium, or other active agents, such as anti-inflammatory agents, anti apoptotic agents, antioxidants, neurotrophic factors or neuroregenerative or neuroprotective drugs as known in the art, either together in a single pharmaceutical composition, or in separate pharmaceutical compositions, simultaneously or sequentially with the other agents (either before or after administration of the other agents); it is expected that the use of said agents increases the efficiency of the cell regeneration or decreases cell degeneration.

Examples of said other agents or components that may be administered with the pluripotent cells include, but are not limited to: (1) other neuroprotective or neurobeneficial drugs; (2) selected extracellular matrix components, such as one or more types of collagen known in the art, and/or growth factors, platelet-rich plasma, and drugs (alternatively, the cells may be genetically engineered to express and produce growth factors); (3) anti- apoptotic agents (e.g., erythropoietin (EPO), EPO mimetibody, thrombopoietin, insulin-like growth factor (IGF)-I, IGF-II, hepatocyte growth factor, caspase inhibitors); (4) anti- inflammatory compounds (e.g., p38 MAP kinase inhibitors, TGF-beta inhibitors, stating, IL-6 and IL-I inhibitors, Pemirolast, Tranilast, Remicade, Sirolimus, and non-steroidal anti-inflammatory drugs (NSAIDS) such as, for example, tepoxalin, tolmetin, and suprofen; (5) immunosuppressive or immunomodulatory agents, such as calcineurin inhibitors, mTOR inhibitors, antiproliferatives, corticosteroids and various antibodies; (6) antioxidants such as probucol, vitamins C and E, coenzyme Q- 10, glutathione, L-cysteine, N- acetylcysteine, etc.; and (7) local anesthetics, to name a few.

The pharmaceutical composition of the invention may be typically formulated as liquid or fluid compositions, semisolids (e.g., gels or hydrogels), foams, or porous solids (e.g., polymeric matrices, composites, calcium phosphate derivatives, and the like, as appropriate for ophthalmic tissue engineering) or particles for cell encapsulation from natural or synthetic origin to allow a better administration of the cells or a higher survival and function. In a particular embodiment, the pluripotent cells may be administered in semi-solid or solid devices suitable for surgical implantation; or may be administered with a liquid carrier (e.g., to be injected into the recipient subject). Thus, the pluripotent cells may be surgically implanted, injected or otherwise administered directly or indirectly to the site of ocular damage or distress. When cells are administered in semi-solid or solid devices, surgical implantation into a precise location in the body is typically a suitable means of administration. Liquid or fluid pharmaceutical compositions, however, may be administered to a more general location in the eye (e.g., intra-ocularly).

The pharmaceutical composition of the invention may be delivered to the eye of a subject (patient) in one or more of several delivery modes known in the art. In an embodiment the pharmaceutical composition is delivered to the retina or surrounding area, via periodic intraocular or intravitrea injection, or under the retina. In addition ideally cells will be delivered only one time at the early onset of the disease, however if there will be a reversion of the phenotype it might be possible additional deliveries during the life of the patient. As it will be understood by a person skilled in the art, sometimes the direct administration of the pharmaceutical composition of the invention to the site wishing to benefit can be advantageous. Therefore, the direct administration of the pharmaceutical composition of the invention to the desired organ or tissue can be achieved by direct administration (e.g., through injection, etc.) by means of inserting a suitable device, e.g., a suitable cannula, or by other means mentioned in this description or known in the technique.

Pharmaceutical compositions for injection may be designed for single-use administration and do not contain preservatives. Injectable solutions may have isotonicity equivalent to 0.9% sodium chloride solution (osmolality of 290-300 milliosmoles). This may be attained by addition of sodium chloride or excipients such as buffering agents and antioxidants, as listed above.

The administration of the pharmaceutical composition of the invention to the subject will be carried out by conventional means, for example, said pharmaceutical composition can be administered to said subject through intravitreal route by using suitable devices such as syringes, cannulas, etc. In all cases, the pharmaceutical composition of the invention will be administered using equipment, apparatuses and devices suitable for administering cell compositions known by the person skilled in the art.

Dosage forms and regimes for administering the pluripotent cells of the invention or any of the other pharmaceutical compositions described herein are developed in accordance with good medical practice, taking into account the condition of the subject, e.g., nature and extent of the retinal degenerative condition, age, sex, body weight and general medical condition, and other factors known to medical practitioners. Thus, the effective amount of a pharmaceutical composition to be administered to a patient will be determined by these considerations as known in the art.

Nevertheless, in general, the pharmaceutical composition of the invention (or any of the other pharmaceutical compositions described herein) will contain a therapeutically effective amount of the pluripotent cells of the invention, preferably a substantially homogenous population of pluripotent cells of the invention to provide the desired therapeutic effect. In the sense used in this description, the term "therapeutically effective amount" relates to the amount of pluripotent cells of the invention which is capable of producing the desired therapeutic effect (e.g., regenerate total or partially the retina and/or rescue of functional vision, and the like) and will generally be determined by, among other factors, the characteristics of said pluripotent cells themselves and the desired therapeutic effect. Generally, the therapeutically effective amount of said pluripotent cells of the invention that must be administered will depend on, among other factors, the characteristics of the subject himself, the seriousness of the disease, the dosage form, etc. For this purpose, the dose mentioned in this invention must only be taken into account as a guideline for the person skilled in the art, who must adjust this dose depending on the aforementioned factors. In a particular embodiment, the pharmaceutical composition of the invention is administered in a dose containing between about 10⁴ and about 10¹⁰ pluripotent cells of the invention per eye, preferably between about 10⁶ and 10⁸ cells per eye. The dose of pluripotent cells of the invention can be repeated depending on the status and evolution of the subject in temporal intervals of days, weeks or months that must be established by the specialist in each case.

In some occasions, it may be desirable or appropriate to pharmacologically immunosuppress a patient prior to initiating cell therapy. This may be accomplished through the use of systemic or local immunosuppressive agents, or it may be accomplished by delivering the cells in an encapsulated device. These and other means for reducing or eliminating an immune response to the transplanted cells are known in the art. As an alternative, the pluripotent cells of the invention may be genetically modified to reduce their immunogenicity.

### Kits

In another aspect, the invention relates to a kit comprising a pluripotent cell of the invention or a pharmaceutical composition of the invention, and instructions for use of the kit components. This kit can be used in the treatment of a retinal degeneration disease.

The particulars of the pluripotent cell of the invention, pharmaceutical composition of the invention, and retinal degeneration disease to be treated have been previously mentioned and are incorporated herein.

### Methods of treatment

According to another aspect of the invention, a method is provided for treating a subject having a retinal degeneration disease (i.e., a patient), which comprises administering to said subject in need of treatment a pluripotent cell of the invention, a pharmaceutical composition made from pluripotent cells of the invention, a pluripotent cell other than the pluripotent cell of the invention, or a pharmaceutical composition made from pluripotent cells other than the pluripotent cells of the invention, in an amount effective to treat the retinal degeneration disease, wherein said treatment of the retinal degeneration disease occurs by reprogramming of retinal neurons mediated by cell fusion of said pluripotent cells with said retinal neurons, said reprogramming being mediated by activation of the Wnt/β-catenin pathway.

The particulars of the pluripotent cells of the invention, pluripotent cells other than the pluripotent cells of the invention, pharmaceutical compositions of the invention, retinal degeneration diseases to be treated and effective amount to treat said diseases have been previously mentioned and are incorporated herein.

In a particular embodiment, the method for treating a subject having a retinal degeneration disease comprises the administration of a pharmaceutical composition made from pluripotent cells of the invention, in an amount effective to treat the retinal degeneration disease, wherein said treatment of the retinal degeneration disease occurs by reprogramming of retinal neurons mediated by cell fusion of said pluripotent cells with said retinal neurons, said reprogramming being mediated by activation of the Wnt/β-catenin pathway.

In another particular embodiment, the method for treating a subject having a retinal degeneration disease comprises the administration of a pharmaceutical composition made from pluripotent cells other than the pluripotent cells of the invention, in an amount effective to treat the retinal degeneration disease, wherein said treatment of the retinal degeneration disease occurs by reprogramming of retinal neurons mediated by cell fusion of said pluripotent cells with said retinal neurons, said reprogramming being mediated by activation of the Wnt/β-catenin pathway.

The present invention is further illustrated, but not limited by, the following examples.

### EXAMPLE 1

### Haematopoietic stem cell fusion triggers retinal regeneration in a mouse model of Retinitis Pigmentosa

### 1. Methods

### Cell preparation

Lineage-negative HSPCs were isolated from total bone marrow of Cre-RFP mice (mice stably expressing CRE and red fluorescent protein [RFP]; provided by Jackson Laboratories) using Lineage Cell Depletion kits (Miltenyi Biotech). They were treated either with 1 µM BIO or PBS and with 1 µM tamoxifen for 24 h before transplantation.

### Animals

**R26Y^{rd1} mice** (mice carrying the R26Lox-Stop-Lox-YFP transgene and homozygous for the *rd1* mutation) [Srinivas et al., BMC Dev Biol 1, 4 (2001)].

### Transplantation

A range of 10⁵-10⁶ cells were transplanted in mice previously anesthetized with an intraperitoneal injection of ketamine: metomidine (80 mg/kg: 1.0 mg/kg, i.p.), the eye lid opened carefully, a small incision made below the ora serrata and 1 up to 5 µl of a solution containing cell suspension in PB S was injected into the vitreus or in the subretinal space. The capillary was maintained in the eye for about 3 seconds to avoid reflux.

### Hybrid FACS sorting

For gene expression analysis, 24 h after cell transplantation mouse retinal tissues were isolated and disaggregated in trypsin, by mechanical trituration. A FACS cell sorter was used to isolate the red and green positive hybrid cells. Total RNA was extracted using RNA Isolation Micro kits (Qiagen), according to the manufacturer protocol. The RNA was reverse-transcribed with SuperScript III (Invitrogen) and qRT-PCR reactions using Platinum SYBR green qPCix-UDG (Invitrogen) were run in an ABI Prism 7000 real-time PCR machine. All experiments were performed in triplicate, and differences in cDNA input were compensated for by normalisation to expression of GAPDH. The primers used in the qRT-PCR analysis are shown in Table 1.

**Table 1**

| **Mouse specific primers for qRT-PCR** | | |
|---|---|---|
| **Species/Gene** | **Sequence 5'-3'** | **SEQ ID NO:** |
| *Gatal* | SuperArray Bioscience Corporation [Catalog No. PPM24651A-200] | |
| *Rhodopsin* | Fw GTAGATGACCGGGTTATAGATGGA | 1 |
| | Rv GCAGAGAAGGAAGTCACCCGC | 2 |
| *RDS* | Fw CGGGACTGGTTCGAGATTC | 3 |
| | Rv ATCCACGTTGCTCTTGCTGC | 4 |
| *Crx* | Fw ATCCGCAGAGCGTCCACT | 5 |
| | Rv CCCATACTCAAGTGCCCCTA | 6 |
| *Rx* | Fw GTTCGGGTCCAGGTATGGTT | 7 |
| | Rv GCGAGGAGGGGAGAATCCTG | 8 |
| *Chx10* | Fw ATCCGCAGAGCGTCCACT | 9 |
| | Rv CGGTCACTGGAGGAAACATC | 10 |
| *Nestin* | Fw TGGAAGTGGCTACA | 11 |
| | Rv TCAGCTTGGGGTCAGG | 12 |
| *Noggin* | Fw CTTGGATGGCTTACACACCA | 13 |
| | Rv TGTGGTCACAGACCTTCTGC | 14 |
| *Otx2* | Fw GAGCTCAGTCGCCACCTCTACT | 15 |
| | Rv CCGCATTGGACGTTAGAAAAG | 16 |

| | | |
|---|---|---|
| [Fw: Forward; Rv: Reverse] | | |

### TUNEL assay

Apoptotic nuclei were detected by TdT-mediated dUTP terminal nick-end labeling kit (TUNEL, fluorescein; Roche Diagnostics, Monza, Italy) according to the producer's protocols.

### H&E staining

Briefly, tissue sections were stained with Histo•Perfect™ *H&E Staining Kit*™. (Manufacturer: BBC Biochemical) according to the producer's protocols.

### Samples treatment

Tissues were fixed by immersion in 4% paraformaldehyde overnight, and then embedded in OCT compound (Tissue-Tek). Horizontal serial sections of 10-mm thickness were processed for analysis. For fluoresceine immunostaining, the primary antibodies used were: anti-Nestin (1:300, Abcam), anti-Otx2 (1:200, Abcam), anti-Noggin (1:200, Abcam), anti-Thy1.1 (1:100, Abcam), anti-syntaxin (1:50, Sigma), anti-glutamine synthetase (Sigma, 1:100) anti-Annexin V (1:200, Abcam) and anti-Ki67 (Sigma, 1.100). The secondary antibodies used were: anti-mouse IgG and anti-rabbit IgG antibodies conjugated with Alexa Fluor 488, Alexa Fluor 546 or Alexa Fluor 633 (1:1000; Molecular Probes, Invitrogen).

### Statistical analysis

The numbers of immunoreactive or YFP-positive cells within three different retinal areas (40× field) were counted in individual sections. A total of 10 serial sections were examined for each eye, from at least three different mice. For statistical analysis, the data were expressed as means ±SEM, as pooled from at least three independent experiments, each carried out in duplicate.

### 2. Results

Retinitis Pigmentosa is a devastating blindness disorder that arises from different mutations in more than 100 known genes [Wright et al. Nat Rev Genet 11, 273-284, doi:nrg2717 [pii]10.1038/nrg2717 (2010)]. *Rdl* mice carry a spontaneous recessive mutation in the *PDE6B* gene that encodes the β subunit of cyclic GMP-specific 3',5'-cyclic phosphodiesterase. This loss of function mutation results in accumulation of cyclic GMP and Ca²⁺ in the rods, which in turn leads to photoreceptor cell death [Doonan et al. Invest Ophthalmol Vis Sci 46, 3530-3538, doi:46/10/3530 [pii] 10.1167/iovs.05-0248 (2005)]. *Rdl* mice are homozygous for this mutation, and they represent a severe model for fast progression of this degenerative disease.

HSPCs are multipotent cells that can give rise to all types of blood cells. In addition, they have been proposed to retain some plasticity with some degree of regenerative potential for different tissues, including for the CNS [Alvarez-Dolado, M. Front Biosci 12, 1-12 (2007)].

Activation of the Wnt/β-catenin pathway has been shown to promote proliferation and dedifferentiation of Müller glia in different mouse models of retinal degeneration, suggesting a possible contribution of this pathway in the modulation of CNS plasticity [Osakada, F. et al. J Neurosci 27, 4210-4219 (2007)]. Indeed, inventors recently reported that periodic activation of the Wnt/β-catenin pathway via Wnt3a or via the GSK-3 inhibitor BIO in embryonic stem cells (ESCs) strongly enhances the reprogramming of neural precursor cells after cell fusion [Lluis et al. Cell Stem Cell 3, 493-507 (2008)]. Inventors, therefore, asked whether fusion of HSPCs with retinal neurons along with a transient activation of the Wnt/β-catenin pathway in transplanted HSPCs might be a mechanism for retinal regeneration and functional vision rescue in *rdl* mice.

Thus, inventors transplanted Lin⁻ HSPCs^{CRE/RFP} (isolated from donor mice stably expressing CRE and red fluorescent protein [RFP]) subretinally in the eyes of postnatal day 10 (p10) R26Y^{rdl} mice (carrying the R26Lox-Stop-Lox-YFP transgene and homozygous for the *rdl* mutation) and sacrificed the mice 24 h later. It was expected to observe RFP and yellow fluorescent protein (YFP) double-positive hybrid cells in case of cell fusion (Fig. 1a). Indeed, it was observed a very high number of hybrids (RFP/YFP-positive) in the outer nuclear layer (ONL) of the retina, and some in the inner nuclear layer (INL) (Fig. 2a).

Inventors have previously shown that the GSK-3 inhibitor BIO does not increase the fusion efficiency of ESCs with neural progenitor cells *in vitro* [Lluis *et al.* (2008) cited *supra*]. Similarly, here it was observed comparable levels of hybrids in the ONL when inventors transplanted HSPCs^{CRE,RFP} pre-treated with BIO for 24 h (henceforth referred to as BIO-HSPCs), to activate the Wnt/β-catenin pathway (Fig. 1b and 1c). This ruled out a role for BIO in modulating fusion efficiency *in vivo*. In contrast, it was not observed any fusion event after subretinal transplantations in control wild-type R26Y mice at p10 (Fig. 1d and 1e), showing that the genetic cell damage triggers fusion between retinal neurons and HSPCs.

HSPCs^{CRE} (not RFP positive) were then transplanted subretinally in R26^{rdl} mice to identify the retinal cell fusion partners. These HSPCs fused specifically with rods in the ONL (rhodopsin/YPF double-positive cells) (Fig. 2b) and with Müller cells (glutamine synthetase/YFP double-positive cells) (Fig. 2c). However, fusion between these HSPCs and cones was never observed (Fig. 2d).

Neurodegeneration in *rdl* mice is already apparent at p10 as the photoreceptors (first rods, and later, as a consequence, cones) undergo apoptosis and degeneration; by p20 these are already almost completely gone. Interestingly, the number of apoptotic photoreceptors decreased substantially after BIO-HSPCs transplantation, which suggested that rod-cell death was delayed or stopped already at 24 h after transplantation (Fig. 2e). Furthermore, in the YFP-positive hybrids that derived from fusion of the BIO-HSPCs^{CRE,RFP} with retinal neurons (i.e., the BIO-hybrids), there were low levels of apoptosis (20%, of total YFP-positive cells) and a high proliferation rate (16%). In contrast, in the hybrids formed between non-BIO-treated HSPCs^{CRE,RFP} and retinal neurons (i.e., no-BIO-hybrids), there were high levels of cell death (75%) and a low proliferation rate (2%) (Fig. 2f, 2g and Fig. 3).

To characterise the YFP/RFP hybrids, they were FACS sorted from the transplanted retinas and analysed for expression of several precursor neuronal and retinal markers, by qRT-PCR analysis (Fig. 2h). The neuronal precursors Nestin, Noggin and Otx2 were clearly activated in the BIO-hybrids, with low activation of the Crx, Rx and Chx10 photoreceptor precursor markers. Moreover, rhodopsin and pheripherin (rds), which are expressed in terminally differentiated photoreceptors, and GATA-1, an HSPC marker, were strongly down-regulated in the BIO-hybrids. In contrast, in the no-BIO-hybrids, there was no reactivation of precursor cell markers or silencing of lineage genes (Fig. 2h).

The protein expression was then analysed in sections. Here, the BIO-hybrids had activated expression of Nestin, Noggin and Otx2; in contrast, in the no-BIO-hybrids, there was almost no activation of these markers (Fig. 4). These data thus show the induction of a dedifferentiation process in the newly generated BIO-hybrids.

In conclusion, BIO-hybrids derived from fusion of the BIO-treated HSPCs with retinal neurons do not enter into apoptosis, but instead undergo cell proliferation and dedifferentiation reactivating different retinal precursor neuronal markers. In contrast, the hybrids derived from non-BIO-treated HSPCs do not proliferate, and nor do they dedifferentiate; instead, they undergo apoptosis.

Next, to investigate whether these BIO-hybrids can regenerate retinal tissue, inventors performed a time-course experiment. BIO-HSPCs^{RFP/CRE} and no-BIO-HSPCs^{RFP/CRE} were transplanted subretinally at p10 in different groups of R26Y^{rdl} mice, and TUNEL and H&E staining were performed on retinal sections after 5 (p15), 10 (p20) and 15 (p25) days, and after 2 months (p60). Although the photoreceptors were still clearly present at p15 in retinal sections from eyes transplanted with both BIO-HSPCs and no-BIO-HSPCs, as shown by the normal structure of the ONL (Fig. 5a, 5b and 6a), the viabilities of the retinal neurons were very different. At p15, there was widespread apoptosis in the photoreceptor layer in sections from the eyes transplanted with no-BIO-HSPCs (Fig. 5c and 6a); in contrast, cell death was almost absent at p15 in the ONL of retinas transplanted with BIO-HSPCs (Fig. 5d and 6a). Remarkably, at the subsequent time points (p20 and p25), the photoreceptor layer in BIO-HSPC-transplanted eyes maintained its normal structure (Fig. 5f, 5h and 6a), while rods and cones nuclei were absent in the ONL of no-BIO-HSPC-transplanted eyes. In their place, few aberrant nuclear layers of cells were seen, which expressed pigmentum and which were positive to the retinal pigment epithelium marker, Rpe65 and to the RFP only (Fig. 5e, 5f, 5g, 6a and 6b).

Finally, at 2 months after transplantation, the retinas of the BIO-HSPCs-transplanted *rdl* mice were still indistinguishable from the wild-type retinas along the entire tissue (Fig. 5i, 5j, 5k and 5l), with 10 rows of photoreceptor nuclei and normal outer and inner segment structures. On the other hand, the histology of no-BIO-HSPCs-transplanted retinas was comparable to those of the non-transplanted *rdl* eyes, with fully degenerated photoreceptor layers (Fig. 5m, 5n, 5o and 5p).

Thus, it can be concluded that the transplanted BIO-HSPCs fully preserved the photoreceptor layer in the *rdl* mouse retinas at least up to two months after their transplantation. This would suggest either a block in the degeneration mechanism or activation of a regeneration process. In contrast, transplantation of no-BIO-HSPCs did not rescue the *rdl* mouse phenotype, even if the transplanted cells retained a moderate potential to transdifferentiate into retinal pigmented epithelium cells.

To investigate differentiation of the hybrids in the long term, R26Y^{rdl} mice were transplanted at p10 with BIO-HSPCs^{CRE} or no-BIO-HSPCs^{CRE} and analysed again two months after the transplantation. Here, there was a full layer of YFP-positive cells in the BIO-HSPC-transplanted *rdl* mouse retinas (Fig. 7a).

Immunofluorescence staining showed that YFP hybrids were differentiated into rods, but not into cones, as they were positive to staining for rhodopsin (Fig. 7a) but not for cone opsin (Fig. 7b). Furthermore no YFP hybrid cells that were also positive for the Müller cell marker glutamine synthetase or for the endothelial cell marker CD31 were found, thus excluding differentiation of the hybrids into Müller cells or into retinal vessels (Fig. 7c and 7d). In contrast, with the no-BIO-HSPC-transplanted cells, almost no YFP-positive hybrids were found two months after their transplantation because the hybrids did not survive for this length of time (Fig. 8a). Thus, it can be concluded that the BIO-hybrids differentiate specifically in rods, and as a consequence the cones are able to survive. All in all, the expression of YFP in all of the rods clearly indicates that newborn hybrids replace the *rdl* mutated photoreceptors, thereby regenerating the retinal tissue.

To further assess hybrid differentiation in rods and to determine whether this fusion-mediated regeneration process can rescue the *rdl* mouse mutation, the expression of *PDE6B*, which is not expressed in *rdl* mice, was analysed. Remarkably YFP/rhodopsin double-positive rods were also positive for *PDE6B* expression, as also confirmed by Western blotting of total extracts from transplanted retina (Fig. 7g). These results indicate that the BIO-hybrids can generate wild-type rods, and thereby can regenerate the retina (Fig. 7e, 7f and 8b); since *rdl* rods cannot express wild-type *PDE6B,* the mutation was complemented by the HSPC genome in the hybrids,

Next, to determine whether regenerated rods were also electrophysiologically functional, inventors performed electroretinogram tests on *rdl* mice 1 month after transplantation of BIO-HSPCs or no-BIO-HSPCs. Of note, both A and B waves under scotopic and photopic conditions were recorded in 4 mice out of 8 transplanted with BIO-HSPCs, with a Δ amplitude in the order of 150 µV on average (not shown) indicating that the regenerated rods underwent cell-membrane hyperpolarisation in response to a light stimulus, and that they could transmit the electric signals to the interneurons, as indicated by the B-wave response. Retinal regeneration under histological analysis confirmed the functional rescue (not shown). Moreover, the visual acuity of a group of treated *rdl* mice between 2.0 and 2.5 months of age were analysed with the optometer test. In the BIO-HSPCs-transplanted *rdl* mice, the number of head tracking movements, which measures the automatic response of the animals when detecting a moving target [Abdeljalil et al. Vision Res 45, 1439-1446, doi:S0042-6989(05)00005-2 [pii]10.1016/j.visres.2004.12.015 (2005)] was significantly higher than that measured in the non-transplanted and no-BIO-HSPCs-transplanted *rdl* mice (not shown). This demonstrated a visual response after stimulus in the BIO-HSPCs-transplanted *rdl* mice.

### 3. Discussion

Some attempts have been undertaken to improve the function of retinal degeneration using bone-marrow-derived stem cells (BMSCs). It has been reported that Lin⁻ HSPCs injected intravitreally in *rdl* mouse eyes can prevent retinal vascular degeneration, a secondary disease phenotype, which then delayed retinal cone degeneration. However, the transplanted retinas were formed of nearly only cones, and the electroretinogram responses were severely abnormal and comparable to untreated animals [Otani et al. J Clin Invest 114, 765-774, doi:10.1172/JC121686 (2004)]. Additional investigations relating to the mechanisms of improved retinal function after BMSC transplantation have been based on the role of BMSCs in promoting an increase in angiogenesis, or a decrease in inflammation, or even anti-apoptotic effects, which might delay retinal degeneration and therefore be beneficial due to slowed progression of the disease. In addition, transdifferentiation of transplanted BMSCs in retinal-pigmented epithelium, which can sustain photoreceptor survival, has been shown in acute eye injury mouse models [Siqueira et al. Arq Bras Oftalmol 73, 474-479, doi:S0004-27492010000500019 [pii] (2010)]. All of these approaches, however, have remained far from therapeutically efficient as they have not been seen to significantly improve the regeneration of retinal tissue.

In addition, systemically transplanted BMSCs have been reported to fuse with resident cells in different tissues, such as heart, skeletal muscle, liver and brain [Terada et al. Nature 416, 542-545 (2002); Alvarez-Dolado et al. Nature 425, 968-973 (2003); Piquer-Gil et al. J Cereb Blood Flow Metab 29, 480-485 (2009)]. However, these fusion events are seen to be very rare, which naturally promotes some skepticism as to their physiological relevance [Wurmser & Gage. Nature 416, 485-487 (2002)]. Here, inventors have clearly demonstrated that if Wnt/β-catenin signalling pathway is not activated, the hybrids undergo apoptosis and therefore cannot be detected at late stages. The majority of these transplanted HSPCs do not fuse, and instead die; however, a few can transdifferentiate into retinal-pigmented epithelium cells, which are of mesenchymal origin. This transdifferentiation can provide some slowing down of the degeneration, but it cannot rescue the phenotype.

In contrast, the activation of the Wnt/β-catenin signalling pathway induces the HSPC genome in the hybrids to activate the *PDE6B* gene; in this condition the hybrids themselves were instructed to differentiate into rods, passing through a transient de-differentiated state. No heterokaryons could be detected, although it cannot formally discarded that there were some present. However, the regenerated photoreceptors co-expressed *PDE6B* and YFP, which indicated that the genomes of the retinal neurons and of the transplanted HSPCs were mixed in the same cells. It remains to be determined whether reduction mitosis or a multipolar mitosis mechanism as previously reported during liver regeneration can reduce the ploidy of the regenerated photoreceptors, or if double genome copies are tolerated in the newborn rods, which finally preserve cone degeneration. Indeed, tetraploid neurons have been identified in mouse and human brain [Wurmser & Gage cited *supra*].

Several gene therapy attempts have been undertaken to treat individual Retinitis Pigmentosa mutations; however, a mutation-independent cell-therapy approach could be much more efficient and practical then creating individual gene therapies to treat each single gene mutation. These data provide real hope for the treatment of patients with Retinitis Pigmentosa as well as further retinal degeneration diseases.

### EXAMPLE 2

### Wnt/β-Catenin Signalling Triggers Neuron Reprogramming in the Mouse Retina

This Example was performed to analyze if somatic cell reprogramming can be induced in tissues in mammalian. The results obtained show that upon activation of the Wnt/β-catenin signalling pathway, mouse retinal neurons can be transiently reprogrammed *in vivo* back to a precursor stage after spontaneous fusion with transplanted haematopoietic stem and progenitor cells (HSPCs). Moreover, it has been shown that retinal damage is essential for cell-hybrid formation *in vivo*. Newly formed hybrids reactivate neuronal precursor markers, *Oct4* and *Nanog*; furthermore, they can proliferate. The hybrids soon commit to differentiation along a neuroectodermal lineage, and finally into terminally differentiated neurons, which can regenerate the damaged retinal tissue. Following retinal damage and induction of Wnt/β-catenin signalling pathway in the eye, cell-fusion-mediated reprogramming also occurs after endogenous mobilisation of bone marrow cells in the eyes. These data show that *in-vivo* reprogramming of terminally differentiated retinal neurons is a possible mechanism of tissue regeneration.

### 1. Experimental Procedures

### Animal care and treatments

All of the procedures on mice were performed in accordance with the ARVO Statement for the Use of Animals in Ophthalmic and Vision Research, and with our institutional guidelines for animal research. All of the animals were maintained under a 12 h light/dark cycle, with access to food and water *ad libitum*.

### Retinal damage and BrdU treatment

Mice at the age of 3 months were anaesthetised by injection of ketamine: metomidine (80 mg/kg: 1.0 mg/kg, intraperitoneal (i.p.)). To induce retinal damage, the animals were treated intravitreally with 2 µl of 20 mM N-methyl-D-aspartate (NMDA) (total 40 nmol; Sigma) for 24 h [Timmers et al., Mol Vis 7, 131-137 (2001)]. Control eyes received 2 µl PBS. For the BrdU incorporation assays, the mice received intraperitoneal (i.p.) BrdU administration of 50 mg/kg body weight.

### Stem cell preparation and transplantation

Retinal stem and progenitor cells (RSPCs) were isolated from the ciliary margin of adult Cre mice as previously described [Sanges et al., Proc Natl Acad Sci USA 103, 17366-17371 (2006)]. Lineage negative HSPCs (Lin⁻ HSPCs) were isolated from the total BM of Cre, Cre-RFP or R26Y mice using Lineage Cell Depletion kits (Miltenyi Biotech). Human CD34⁺ HSPCs were purchased from StemCell Technologies. Cells were pre-treated with 1 µM tamoxifen for 24 h to induce nuclear translocation of Cre recombinase, and labelled with Vybrant DiD (5 µl/ml) (Invitrogen) before transplantation, where necessary.

To obtain ESCs^{Cre}, 5 × 10⁶ ESCs were electroporated with the Cre-recombinase-carrying vector (CAGG-Cre), using ES nucleofector kits (Amaxa).

The stem cells (SCs) were left non-treated or were pretreated with 100 ng/ml Wnt3a or 1 µM BIO, for 24 h, and finally 5 ×10⁵ cells were injected intravitreally into the eyes of the anaesthetised mice. The mice were sacrificed by cervical dislocation, and their eyeballs were enucleated for histological analyses.

### Hybrid isolation for gene expression and tetraploidy analysis

Twenty-four hours after cell transplantation, the retinal tissue was isolated from treated mice and disaggregated in trypsin by mechanical trituration.

To analyze the tetraploid content of hybrids, cells were pelleted, washed twice with 1x PBS and fixed for 2 h in ice with 70% ethanol. After fixation, cells were washed twice with 1x PBS and incubated with 25 µg/ml propidium iodide and 25 µg/ml RNAse A (Sigma-Aldrich) for 30 minutes at room temperature. Samples were analyzed by flow cytometry in a FACSCanto (Becton Dickinson). Doublet discrimination was performed by gating on pulse-width versus pulse-area of the PI channel. Samples were analyzed with FlowJo software (Tree Star, Inc).

For gene expression analysis, a BD FACSAria II sorting machine (Becton Dickinson) was used to isolate the red and green positive hybrids cells. Total RNA was extracted using RNA Isolation Micro kits (Qiagen), according to the manufacturer protocol. The eluted RNA was reverse-transcribed with SuperScript III (Invitrogen) and qRT-PCR reactions using Platinum SYBR green qPCix-UDG (Invitrogen) were performed in an ABI Prism 7000 real-time PCR machine, according to the manufacturer recommendations. The species specific oligos used are listed in Table 2. All of the experiments were performed in triplicate, and differences in cDNA input were 'compensated' normalising to the expression of GAPDH.

**Table 2**

| **Human and mouse specific primers for sRT-PCR** | | |
|---|---|---|
| Human specific primers for qRT-PCR | | |
| **Species/Gene** | **Sequence 5'-3'** | **SEQ ID NO:** |
| *hOct4* | Fw TCGAGAACCGAGTGAGAGGC | 17 |
| | Rv CACACTCGGACCACATCCTTC | 18 |
| *hNanog* | Fw CCAACATCCTGAACCTCAGCTAC | 19 |
| | Rv GCCTTCTGCGTCACACCATT | 20 |
| h*Nestin* | Fw TGTGGCCCAGAGGCTTCTC | 21 |
| | Rv CAGGGCTGGTGAGCTTGG | 22 |
| *hOtx2* | Fw ACCCCTCCGTGGGCTACCC | 23 |
| | Rv CAGTGCCACCTCCTCAGGC | 24 |
| *hNoggin* | Fw AGCACGAGCGCTTACTGAAG | 25 |
| | Rv AAGCTGCGGAGGAAGTTACA | 26 |
| hCD34 | Fw GTTGTCAAGACTCATGAACCCA | 27 |
| | Rv ACTCGGTGCGTCTCTCTAGG | 28 |

| Mouse specific primers for qRT-PCR | | |
|---|---|---|
| **Species/Gene** | **Sequence 5'-3'** | **SEQ ID NO:** |
| m*Oct4* | Fw CGTGGAGACTTTGCAGCCTG | 29 |
| | Rv GCTTGGCAAACTGTTCTAGCTCCT | 30 |
| m*Nanog* | Fw GCGCATTTTAGCACCCCACA | 31 |
| | Rv GTTCTAAGTCCTAGGTTTGC | 32 |
| m*Nestin* | Fw TGGAAGTGGCTACA | 11 |
| | Rv TCAGCTTGGGGTCAGG | 12 |
| mOtx2 | Fw GAGCTCAGTCGCCACCTCTACT | 15 |
| | Rv CCGCATTGGACGTTAGAAAAG | 16 |
| m*Noggin* | Fw CTTGGATGGCTTACACACCA | 13 |
| | Rv TGTGGTCACAGACCTTCTGC | 14 |
| mCD34 | Fw CTGGTACTTCCAGGGATGCT | 33 |
| | Rv TGGGTAGCTCTCTGCCTGAT | 34 |

| | | |
|---|---|---|
| [Fw: Forward; Rv: Reverse] | | |

Gata1 primers were purchased at SuperArray Bioscience Corporation [Catalog number PPM24651A-200]

### Bone marrow (BM) replacement

BM transplantation was conducted as previously reported with minor modifications. The BM of 4- to 6-week-old R26Y or Nestin-Cre recipient mice was reconstituted with BM cells from the tibias and femurs of RFP/CRE or R26Y transgenic mice respectively. BM cells (1x10⁷ cells) were injected intravenously into the recipients 3 hours after irradiation with γ-rays (9 Gy). The eyes of the recipients were protected with lead shields to prevent radiation-induced damage (radiation retinopathy). Four weeks after transplantation, the peripheral blood of chimeric mice was extracted from the tail vein, and the reconstituted BM was assessed.

### Fixing, sectioning and immunohistochemistry

Tissues were fixed by immersion in 4% paraformaldehyde overnight, and then embedded in OCT compound (Tissue-Tek). Horizontal serial sections of 10 µm thickness were processed for immunohistochemistry, and visualisation of Nanog-GFP and Rosa26-YFP fluorescence was performed by fluorescent microscopy.

For fluoresceine immunostaining, the primary antibodies used were: anti-Nanog (1:200, R&D), anti-Oct4 (1:100, AbCam), anti-Nestin (1:300, Abcam), anti-GATA4 (1:500, Abcam), anti-Otx2 (1:200, Abcam), anti-Noggin (1:200, Abcam), anti-Handl (1:400, Abcam), anti-Tuj-1 (1:100, Abcam), anti-Thyl.1 (1:100, Abcam), anti-syntaxin (1:50, Sigma), anti-glutamine synthetase (Sigma, 1:100) anti-Annexin V (1:200, Abcam), anti-Ki67 (Sigma, 1.100) and anti-BrdU (1:300, Sigma). The secondary antibodies used were: anti-mouse IgG and anti-rabbit IgG antibodies conjugated with Alexa Fluor 488, Alexa Fluor 546 or Alexa Fluor 633 (1:1000; Molecular Probes, Invitrogen).

Percentages of GFP and YFP positive cells were evaluated counterstaining the tissue sections with DAPI (Vectashield, Vector Laboratories, Burlingame, CA, USA), and they were photographed using either an Axioplan microscope (Zeiss) or a Leica laser confocal microscope system.

### In-vitro culture of reprogrammed hybrids

BIO-treated or non-BIO-treated ESCs or HSPCs were injected into the eyes of NMDA-damaged Nanog-GFP-puro mice. Twenty-four hours after transplantation, the retinal tissue was isolated and treated with trypsin for 30 min at 37°C. The cells were then resuspended as single-cell suspensions in ES culture media using a fire bore hole Pasteur, and plated onto gelatine-coated dishes at a concentration of 3 ×10⁵ cells/ 9.6 cm². To select the reprogrammed clones, puromycin was added to the culture medium after 72 h. GFP-positive clones were counted and photographed after one month of culture. The clones were alkaline phosphatase (ΔP) stained after 1 month of culture, as previously described [Lluis et al., Cell Stem Cell 3, 493-507 (2008)].

### Statistical analysis

The numbers of immunoreactive or of Nanog-GFP- and -YFP-positive cells within three different retinal areas (40× field) were counted in individual sections. A total of ten serial sections were examined for each eye, from at least three different mice. For statistical analysis, the data were expressed as means ±SEM, as pooled from at least three independent experiments, each carried out in duplicate. The experiments were performed using at least three different mice. Differences were examined using the unpaired Student t-test.

### 2. Results

### NMDA-induced injury triggers fusion between retinal neurons and stem cells

Although cell fusion-mediated somatic cell reprogramming can be induced *in culture*, it remained to be seen if terminally differentiated cells can be reprogrammed via cell fusion within tissues of adult vertebrates.

Thus, inventors first determined whether SPCs could fuse with retinal neurons *in vivo*. For this, inventors used transgenic mice carrying YFP flanked by loxP sites under the control of the ubiquitously expressed Rosa26 promoter as recipients (i.e. with a LoxP-STOP-LoxP-YFP [R26Y] allele) [Srinivas et al., BMC Dev Biol 1, 4 (2001)]. Different SPCs stably expressing Cre recombinase and labelled in red were transplanted in the eyes of recipient mice by intra-vitreal injection (5 ×10⁵ cells/eye). Specifically, inventors used Lineage negative (Lin⁻) HSPCs^{Cre/RFP} isolated from CRE-RFP double transgenic donor mice, 1,1'-dioctadecyl-3,3,3',3'-tetramethylindodicarbocyanine dye (DiD)-labelled RSPCs^{Cre} isolated from the ciliary margin of Cre transgenic mouse eyes [Sanges et al., Proc Natl Acad Sci U S A 103, 17366-17371 (2006)], and DiD-labelled ESCs^{Cre} generated by the inventors. Mice were sacrificed at different times after SPCs injection. If cell fusion had occurred between the injected SPCs^{Cre} and LoxP-STOP-LoxP-YFP (R26Y) retinal neurons, it could be expected to detect YFP expression in retinal sections, due to excision of the STOP codon by Cre (Fig. 9A).

Firstly, inventors tested whether retinal tissue damage caused by intravitreal injections of NMDA in R26Y mice could induce cell fusion. NMDA caused apoptosis of neurons in the inl and gcl of the retina (Fig. 10A and 10B), as shown previously [Osakada et al., J Neurosci 27, 4210-4219 (2007)]; however, NMDA did not enhance the stochastic expression of the YFP transgene in these R26Y mice (Fig. 10C). Then, inventors induced NMDA damage in the right eye of R26Y mice and left the contralateral eye undamaged as control; 24 h later HSPCs^{Cre/RFP} were transplanted into both eyes. Mice were finally sacrificed 24, 48 or 72 h after transplantation (Fig. 9A).

Already 24 h after transplantation, up to 70% of the injected HSPCs^{Cre/RFP} that were detected in the optical field had fused with retinal cells, thus giving rise to YFP-positive hybrids (Fig. 9B, 9D and 10D). Interestingly, the transplanted cells integrated into the retinal tissue and crossed the gcl, even reaching the inl (Fig. 9B, NMDA). In contrast, there were no YFP-positive hybrids in the controlateral, non-damaged, eyes; furthermore, transplanted HSPCs^{Cre/RFP} remained on the border of the gcl and were not integrated into the retinal tissue (Fig. 9C and 9D, No NMDA). Similar results were seen in retinal sections of mice sacrificed 48 and 72 h after transplantation (Fig. 9D). The presence of tetraploid cells was also analysed by flow cytometry. Nuclei with 4C DNA content were clearly evident in the hybrids present in the retinas of R26Y mice transplanted with HSPCs^{Cre/RFP} (Fig. 10C).

These data demonstrated that the injury was necessary to induce migration of transplanted HSPCs into the retinal tissue and their fusion with retinal neurons.

The localization of the hybrids (YFP positive cells) in the retinal tissue suggested that transplanted cells fused with ganglion cells (that localize their nuclei in the gcl) and amacrine cells (that localize across the inl and the inner plaxiform layer (ipl)) (Fig. 10A); to note, those are the retinal cells specifically damaged after NMDA treatment [Osakada *et al.* (2007), cited *supra*]. Thus, to confirm which of the retinal cells fused with the HSPCs, inventors analyzed the expression of different retinal cell markers in the YFP-positive hybrids 12 h after the transplantation of Lin⁻ HSPCs^{Cre/RFP} into NMDA-damaged R26Y eyes. YFP hybrids either positive to the ganglion-cell marker thyl.1 in the gcl (Fig. 9E), or to the amacrine-cell marker syntaxin in the ipl (Fig. 9F). No co-localisation was seen with the Müller cell marker glutamine synthetase (Fig. 9G). 60% of the YFP-positive hybrids were thy1.1-positive, while 22% were syntaxin positive (Fig. 10F and 10F), indicating that the majority of the hybrids were formed between ganglion cells and HSPCs, with some fusion with amacrine cells. In the remaining 18% of the YFP-positive hybrids, the fusion partners were unclear; indeed, there might also have been down-regulation of thy1.1 and/or syntaxin in the newly formed hybrids.

Next inventors performed similar experiments by injecting DiD-labelled ESCs^{Cre} and DiD-RSPCs^{Cre} into undamaged and NMDA-damaged eyes of the R26Y mice. With both cell types, up to 70% of the injected cells detected in the optical field fused with retinal neurons (Fig. 11A, 11B, and 10D). Also these cells fuse with ganglion and amacrine retinal neurons as demonstrated by the localisation of the YFP signal in the gcl and ipl and by the co-localisation of the YFP and thyl.l or syntaxin signals (Fig. 11A, 11B and data not shown).

To further confirm that injected SPCs do indeed fuse with post-mitotic retinal neurons, the proliferative potential of retinal cells before the fusion event was analyzed. Concurrently inventors injected thymidine analogue 5'-bromo-2'-deoxyuridine (BrdU) intraperitoneally and NMDA into the eyes of R26Y mice; then, after 24 h ESCs^{Cre} were injected and, finally, the mice were sacrificed 24 h after this transplantation. Not BrdU-positive cells (red arrows) were seen to be also positive for YFP (green arrows), thus excluding the fusion of ESCs with proliferating cells (Fig. 11C). The BrdU-positive cells found next to the gcl (Fig. 11C, red arrows) were probably microglial cells that had been recruited to the retina following the damage [Davies et al., Mol Vis 12, 467-477].

Overall, these data demonstrate that HSPCs, ESCs and RSPCs can spontaneously fuse with retinal neurons *in vivo* upon cell damage.

### The Wnt/β-catenin signalling pathway triggers reprogramming of retinal neurons in vivo

Wnt/β-catenin signalling pathway is activated after NMDA damage resulting in increased expression of β-catenin, which accumulates into the cells (see Fig. 12A and Osakada *et al.* (2007) cited *supra*). Thus, inventors tested whether endogenous Wnt/β-catenin pathway activation could mediate reprogramming after cell fusion *in vivo*.

For this, two different mouse models were used as recipient mice: Nestin-CRE (transgenic mice expressing Cre recombinase gene under the control ofNestin promoter in neural precursors) [Tronche et al., Nat Genet 23, 99-103 (1999); Okita et al., Nature 448, 313-317 (2007)]and Nanog-GFP-Puro mice (transgenic mice expressing GFP-puromycine genes under the control of the Nanog promoter in the embryo [Okita et al., Nature 448, 313-317 (2007)], which allowed us to investigate reprogramming at the neuronal precursor and the embryonic stages, respectively. DiD-labelled HSPCs^{R26Y} and HSPCs^{RFP} were injected into the eyes of the Nestin-CRE and Nanog-GFP-puro mice, respectively. NMDA was injected intravitreally into one eye of a group of mice, while the contralateral eye remained undamaged as control. Importantly here, no expression of Nanog-GFP (Fig. 12C) or Nestin-Cre (data not shown) transgene was detected following NMDA treatment in the ganglion and amacrine cell. After 24 h, HSPCs were injected into both the non-treated and NMDA-treated eyes, and the mice were sacrificed after an additional 24 h. In the case of reprogramming of the retinal neurons, in these mouse models it could be expected to find double red/green positive cells (Fig. 13A and Fig. 12B). No green-positive cells were seen after injection of HSPCs into the non-damaged eyes (Fig. 13B, 13C, 13D and 13E, No NMDA). In contrast, about 30% and 20% of the total red cells were also green when HSPCs were injected into the NMDA-damaged eyes of Nestin-CRE and Nanog-GFP-puro mice, respectively (Fig. 13B, 13C, 13D and 13E, NMDA; and 10C), indicating that up to 30% of the hybrids were indeed reprogrammed, as they had reactivated Nanog and Nestin promoters in the neuron genome.

To assess the role of activation of the endogenous Wnt/β-catenin signalling pathway in the reprogramming of retinal neurons, in both of these mouse models, DKK1 was also injected immediately after NMDA injection; DKK1 is an inhibitor of the Wnt/β-catenin pathway [Osakada et al. (2007) cited *supra*, Fig. 12A]. HSPCs were transplanted after 24 h, and mice were sacrificed 24 h later. DKK1 injections almost completely blocked the reprogramming of neuron-HSPC hybrids (Fig. 13B, 13C and 13D, NMDA+DKK1), which demonstrated that endogenous and damage-dependent activation of the Wnt/β-catenin pathway triggers reprogramming of retinal neurons after their fusion with HSPCs.

Next, inventors aimed to analyse whether reprogramming of retinal neurons was increased after transplantation of HSPCs in which the Wnt/β-catenin signalling pathway had been previously activated by the GSK-3 inhibitor BIO or by Wnt3a treatment before injection (Fig. 12D, 12E and 12F). Surprisingly, 24 h after transplantation of BIO-pretreated or Wnt3a-pretreated HSPCs in NMDA-damaged eyes of the Nestin-CRE and Nanog-GFP mice, there was a striking increase in the number of reprogrammed (green-positive) hybrids with respect to those seen in NMDA-damaged eyes that received untreated-HSPCs (Fig. 13B and 13E; NMDA+BIO, NMDA+Wnt3a). Similar results were also observed in mice sacrificed 48 h and 72 h after cell transplantation (data not shown). Of note, after injection of BIO-treated HSPCs into non-damaged eyes, both Nanog-GFP-puro (Fig. 12G) and Nestin-CRE transgenes (data not shown) were not expressed, confirming that the tissue damage is necessary for spontaneous cell fusion-mediated retinal neuron reprogramming.

To evaluate the efficiency of this *in-vivo* reprogramming, inventors counted the green-positive reprogrammed cells relative to the total population of red-HSPCs detected in the optical field (Fig. 10C). In the damaged retinas, up to 65% of both the BIO-pretreated and the Wnt3a-pretreated HSPCs reprogrammed retinal neurons after fusion, leading to the formation of double-positive HSPC-neuron hybrids in both of these mouse models (Fig. 13C and 13D).

Given that it was surprising to observe reprogramming at the embryonic stage after fusion of HSPCs with terminally differentiated neurons, it was investigated activation of the Nanog-GFP transgene after transplantation of ESCs and RSPCs into NMDA-injured eyes.

As it could be expected, in the case of the ESC transplantation, reprogramming of the retinal neurons, which was also dependent on activation of the endogenous Wnt/β-catenin signalling pathway, was also observed. GFP-positive cells were also strikingly increased when ESCs were pretreated with BIO or with Wnt3a before being transplanted (Fig. 14A). To confirm reprogramming of ESC-retinal neuron hybrids, inventors cultured GFP-positive hybrids FACS-sorted from NMDA-damaged retinas of Nanog-GFP-puro mice transplanted with BIO-treated or untreated ESCs. Reprogrammed GFP positive colonies were grown *in culture* and they were also resistant to puromycine selection and positive to the alkaline phosphatise (AP) staining (Fig. 14B).

In contrast, no reprogramming events after injection of RSPCs into the NMDA-injured eyes of the Nanog-GFP mice was observed, even in the case of BIO pre-treatment of transplanted RSPCs (Fig. 14C). Interestingly, only a few YFP-positive cells were observed after transplantation of BIO-treated RSPC^{R26Y}, in NMDA-damaged eyes ofNestin-CRE mice (not shown).

Finally, it was also ruled out an effect of BIO in the enhancement of fusion events *in vivo.* BIO pre-treatment did not enhance the fusion efficiency of the HSPCs, ESCs or RSPCs injected into the NMDA-damaged eyes of the R26Y mice (Fig. 14D).

In conclusion here, it has been shown that activation of the Wnt/β-catenin signalling pathway triggers the reprogramming of retinal neurons back to an embryonic/neuronal precursor stage, and that this occurs following damage-dependent cell-cell fusion of HSPCs and ESCs, but not of RSPCs.

To better characterize the reprogrammed hybrids, in addition to look at Nestin-CRE and Nanog-GFP transgenes reactivation, the expression profile of precursor and embryonic genes *in vivo* was analyzed in the newly formed hybrids. Then it was injected BIO-treated or untreated HSPCs^{Cre/RFP} in NMDA-damaged eyes of R26Y mice, and 24 h later the hybrid cells from the retinas were sorted by FACS. Marker expression was analysed by real time PCR. In the BIO-hybrids (hybrids formed by the BIO-treated HSPCs) Oct4, Nanog, Nestin, Noggin and Otx2 were up-regulated (Fig. 15A); indeed, no expression of these genes was detected in the non-transplanted NMDA-injured retinas, nor in the HSPCs, with the exception of Nanog in the HSPCs (Fig. 16A and 16B). In contrast, Gata1, which is a HSPC specific gene, was down-regulated in the BIO-hybrids (Fig. 15A). None of the precursors markers were re-expressed (or they were poorly expressed in the case of Nanog and Nestin) in non-BIO-hybrids, where expression of Gata1 was comparable to that observed in the HSPCs (Fig. 15A and Fig. 16B).

Expression of Oct4, Nanog and Nestin proteins in the BIO-hybrids was also confirmed by their merged immunostaining signals with the YFP-positive hybrids in sections (Fig. 15B).

However, to clearly demonstrate that the re-expression of embryonic and progenitor markers resulted from reprogramming of the neuron genome and not from the genome of the injected HSPCs, inter-species hybrids were analyzed. For this, inventors transplanted BIO-treated and DiD-labelled CD34⁺ human HSPCs into damaged eyes of Nanog-GFP mice. The reprogramming of the retinal neurons was confirmed in sections after fusion of the human HSPCs (Fig. 16C). Interestingly Oct4, Nanog, Nestin, Noggin and Otx2 were all expressed (as analysed with mouse-specific oligonucleotides; see Table 2) from the reprogrammed mouse neuron genome in the sorted hybrids (Fig. 15C). In addition, in the human genome, expression of Oct4, Nestin, Otx2 and Noggin was activated, while CD34 was down-regulated (Fig. 15D).

Overall, it can be concluded that HSPC-fusion-mediated reprogramming of retinal neurons controlled by Wnt/β-catenin signalling pathway can occur *in vivo*.

### Reprogrammed neurons can proliferate and differentiate in vivo

Next the proliferative potential of the reprogrammed neurons was investigated. Thus, BIO-treated and untreated HSPCs^{Cre} were injected into the NMDA-damaged eyes of a group of R26Y mice and retinal sections were analysed 24 h later.

Surprisingly, 8% of the YFP-positive reprogrammed hybrids (after injection of BIO-treated HSPCs^{Cre}) underwent proliferation (Fig. 15E and 15G, Ki67/YFP double positive); these cells were not committed to an apoptotic fate, as only about 5% of the hybrids were positive for Annexin-V staining (Fig. 15F and 15H). On the contrary, injection of non-BIO-treated HSPCs^{CRE} led to the formation of non-proliferative hybrids (Fig. 15E and 15I; as negative to Ki67 staining) that underwent apoptosis, as up to 30% of the YFP-positive hybrids were positive for Annexin-V staining (Fig. 15F and 15J, Anexin-V/YFP double positive). Similar results were obtained 72 h after transplantation of BIO-treated or non-BIO-treated ESCs (Fig. 16D and 16E). Overall, these data show that hybrids formed between HSPCs or ESCs and retinal neurons embark into apoptosis, but if the Wnt/β-catenin pathway is activated in the transplanted SPCs, the neurons can be reprogrammed, survive and re-enter into the cell cycle.

Inventors then analysed the *in-vivo* differentiation potential of the reprogrammed retinal neurons in the NMDA-damaged retinas of the R26Y mice injected with BIO-treated and non-BIO-treated HSPCs^{Cre}. The mice were sacrificed 24, 48 and 72 h after transplantation. The percentages of YFP-positive hybrids for each marker were determined from retinal sections (Fig. 10C). Remarkably, 24 h after the injection of the BIO-treated HSPCs, reprogrammed neurons (as YFP positive) were already re-expressing Nestin, Noggin and Otx2, and this expression was maintained at the subsequent time points analysed (48, 72 h). Conversely, the neuronal terminal differentiation marker Tuj-1 was progressively silenced. In addition Sca1 and c-kit were strongly down-regulated in these hybrids. Oct4 and Nanog were also highly expressed at 24 and 48 h, although their expression was decreased by 72 h (Fig. 15B and 15K). In contrast, a low number of YFP positive hybrids obtained after fusion with non-BIO-treated HSPCs reactivated Nestin, Noggin and Otx2, and instead they maintained expression of Tuj-1, Sca-1 and c-kit in the majority of the hybrids. Oct4 and Nanog were also expressed, although only at 24 h and in very few hybrids at the later (48, 72 h) time points (Fig. 15L), GATA4, a mesoderm marker, and Hand1, an endoderm marker, were never expressed in the hybrids (Fig. 15K and 15L). In conclusion, the BIO-hybrids were reprogrammed and tended to differentiate into the neuroectoderm lineage. In contrast, the non-BIO hybrids were poorly reprogrammed, and thus they did not embark into neuroectoderm differentiation.

Similar differentiation analysis, performed after injection of BIO-treated ESCs^{Cre} in the damaged R26Y retinas, showed a delay in the neuroectodermal differentiation potential of the resulting hybrids (as Nestin, Noggin and Otx2 were expressed more at 72 h after ESC injection while Oct4 and Nanog where highly expressed from 24 to 72 h) and the positive expression of Gata4 and Hand1. These results indicate that ESC-neuron hybrids are more pluripotent and can differentiate in the neuroectodermal lineage but also in the mesoderm and endoderm lineages (Figure 16F).

Finally, inventors investigated whether the BIO-hybrids that were committed to a neural differentiation fate can terminally differentiate into retinal neurons and thus regenerate the damaged retina. For this, a group of NMDA-damaged eyes of R26Y mice were injected with BIO-treated or non-BIO-treated HSPCs^{Cre/RFP} and sacrificed 2 weeks later. Remarkably, YFP/RFP neurons in the gcl and in the inl were observed only after transplantation of BIO-treated HSPCs (Fig. 17A, 17B and 17C). These cells were positive to the markers for thy1.1 and syntaxin, clearly indicating that the hybrids differentiated into ganglion and amacrine neurons (Fig. 18A and 18B). On the contrary, no YFP/RFP hybrids were detected 2 weeks after transplantation of untreated HSPCs (Figure 17D, 17E and 17F), as they undergo cell death at short time. Next, the histology of the transplanted retina was analyzed. Strikingly, it was observed that the number of nuclear rows in the gcl and in the inl of the retinas transplanted with the BIO-treated HSPCs was increased substantially with respect to those with the non-BIO-treated HSPCs and to untransplanted retinas. Their numbers were comparable to those in the wild-type retina (Fig. 17G, 17H, 17I, 17J, 17K and 17L). These data clearly indicate that reprogrammed HSPC-neuron hybrids can differentiate in retinal neurons and regenerate the damaged retina. Thus, it can be concluded that cell-fusion-mediated reprogramming can trigger retinal tissue regeneration.

### Endogenous BMC fusion-mediated reprogramming of retinal neurons occurs in vivo after damage

It has been reported that endogenous BMCs can be recruited into the eye after NMDA damage [Sasahara et al., Am JPathol 172, 1693-1703 (2008)]; however, their role remains unknown. Thus, inventors investigated whether endogenous BMCs can also fuse and reprogramme retinal neurons after NMDA damage. For this, BMCs from donor RFP-CRE mice (transgenic mice expressing RFP and CRE, both under the control of the ubiquitously expressed β-action promoter (Long et al., (2005). BMC Biotechnol 5, 20; Srinivas *et al.* (2001) cited *supra*)) were tail-vein transplanted in sub-lethally irradiated R26Y recipient mice, thereby substituting their BM with BM^{Cre/RFP}. The repopulation of the BM with cells of donor origin was analysed according to the expression of RFP in blood cells and by haemocytometric analysis (Fig. 19A). One month after transplantation, NMDA was injected intra-vitreally in one eye of each group of chimeric mice, and then 24 h later the mice were sacrificed (Figure 20A). Interestingly, it was found that after NMDA damage, 50% of the RFP-positive cells were also YFP positive, indicating fusion of endogenous BMCs recruited in the eyes (Fig. 20B, 20C and 20F, NMDA, and Fig. 10D). In contrast, no RFP/YFP-positive cells were found in sections of non-NMDA-injected eyes (Fig. 20D, 20E and 20F, No NMDA). These results, clearly demonstrate that cell fusion occurs between the BMCs recruited into the eyes and the retinal neurons.

Inventors then analysed the identity of these hybrid cells that were obtained after endogenous cell fusion. It was observed that 12 h after NMDA damage, the YFP-positive cells in the retinal sections were also positive for the Scal, Ckit, Thy1.1, Syntaxin, and GS cell markers, clearly indicating that HSPCs were recruited from the BM and fuse with ganglion, amacrine and Müller cells (Fig. 20G, 20H, 20I, 20J and 20K).

Next inventors investigated if reprogramming can occur after BMC recruitment and fusion with retinal neurons; to this aim, BMCs^{R26Y} were transplanted into a group of sub-lethally irradiated Nestin-CRE mice to generate chimeric mice. The reactivation of Nestin-CRE transgene and the consequent YFP expression enabled us to identify reprogramming events after BMC recruitment in the eye. One month later, NMDA and BIO were injected into only one eye of the chimeric mice, which were sacrificed 24 h later (Fig. 21A). YFP-positive cells were observed after injection of BIO in the gcl and inl of NMDA damaged eye, but not in the NMDA-damaged (non-BIO injected) untreated contralateral eyes (Fig. 21B and 21C). This clearly indicates that the retinal neurons had fused with the recruited BMCs and were reprogrammed because of the reactivation of the Nestin promoter. About 8% of these YFP-positive hybrids were positive for Ki67 expression, and only 1% were Annexin-V positive, which indicated that some of the hybrids were dividing and very few were apoptotic (Fig. 21D, 19B and 19C). Strikingly, 50% of these YFP-positive hybrids were also positive for Oct4 expression (Fig. 21E, 21F and 21G), and 70% for Nanog (Fig. 21E, 21H and 21I), confirming that reprogramming of the retinal neurons occurred also after mobilisation of the BMCs into the eyes.

In conclusion, endogenous activation of BMC-fusion-mediated reprogramming of retinal neurons can occur in the eye if the Wnt/β-catenin pathway is activated.

### 3. Discussion

Here it has been demonstrated that the canonical Wnt/β-catenin signalling pathway mediates the reprogramming of retinal neurons *in vivo*. In addition, it has been shown that spontaneous cell fusion can occur in the mouse retina after injury and that a proportion of fusion hybrids proliferate if they are reprogrammed by Wnt activity. Furthermore, it has also been shown that if not reprogrammed, the neuron-SPC hybrids undergo apoptosis. Surprisingly, the reprogrammed hybrids can regenerate the damaged retinal tissue. Finally, it has been clearly showed that after activation of the Wnt/β-catenin signalling pathway in the eye, BM-derived cells that are recruited into the injured retina can fuse and reprogramme the retinal neurons upon activation of the Wnt/β-catenin signalling pathway. Overall, it can be concluded that cell-fusion-mediated reprogramming can be an endogenous mechanism of damage repair.

Adult SPCs show a high degree of plasticity and pluripotency, and they can contribute to a wide spectrum of differentiated cells. Transplanted BMCs can fuse and acquire the identity of liver cells, Purkinje neurons, kidney cells, epithelial cells, and more. This plasticity has been ascribed to either transdifferentiation or cell-cell fusion mechanisms.

Up to now however, cell fusion events have been considered very rare, and therefore the cell identity of the "newborn" hybrids has never been clearly investigated. Here, inventors have demonstrated that cell-cell fusion occurs and can be visualised as a very relevant event shortly after transplantation of HSPCs into a damaged eye. This is true also after mobilisation of c-kit/sca-1-positive cells from the BM into damaged retinas. In previous studies, the numbers of hybrids derived from BMC fusion have been largely underestimated; indeed, it has been found here that unless these newly formed hybrids are reprogrammed, they undergo cell death, and therefore a long time after the transplantation they cannot be detected.

HSPCs fuse with high efficiency with ganglion and amacrine neurons; the resulting "newborn" hybrids are novel cell entities, which if a Wnt-signalling stimulus is provided, can initially be transiently reprogrammed and can proliferate and then become terminally differentiated neurons. It is remarkable that it was found expression of Nanog and Oct4, and at the same time, expression of Nestin, Noggin and Otx2 precursor neuronal markers in these hybrids. The expression of Nanog and Oct4 is a clear evidence of reprogramming back to the embryonic stage; however, this state is transient, at least in the case of fusion between HSPCs and retinal neurons. The hybrids very soon commit to neuroectodermal lineage, and indeed, 72 h after transplantation, Oct4 and Nanog were already down-regulated. Finally, in two weeks, the hybrids become terminally differentiated neurons and regenerate the gcl and the inl in the retinal tissue. Interestingly, it was also observed full functional regeneration of photoreceptors in a mouse model of Retinitis Pigmentosa (RP) after cell-fusion-mediated reprogramming of retinal neurons upon transplantation of Wnt/β-catenin pathway activated HSPCs (Example 1).

These observations led to anticipate that Oct4 and Nanog are not only stem cell genes that are expressed in embryos, but that they have a functional role also in adult tissue during cell-fusion-mediated regeneration processes. Expression of these genes in adults is controversial [Shin et al., Mol Cells 29, 533-538 (2010); Kucia et al., J Physiol Pharmacol 57 Suppl 5, 5-18 (2006)]; however, it might well be that their expression has not been clearly appreciated in some circumstances, probably due to its very transient nature.

ESCs also have great plasticity, and here inventors were able to identify dedifferentiation events *in vivo*; i.e., reprogrammed hybrids expressing Nanog after the fusion of retinal neurons with ESCs. ESC-retinal-neuron hybrids are probably more pluripotent than HSPC-derived hybrids. They can form clones *in culture* and express markers of three different lineages; in addition, they form teratoma *in vivo* (data not shown). In contrast, *in vitro*, inventors were not able to isolate clones from HSPC-retinal neuron hybrids, clearly indicating their transient reprogramming and fast commitment to neuroectoderm lineage differentiation. Interestingly, reprogramming of retinal neurons up to the expression of Nanog was not observed after fusion of RSPCs, indicating the lower degree of plasticity of these cells with respect to HSPCs.

For a long time, it has been thought that differentiation is a one-way-direction mechanism; the possibility to induce somatic-cell reprogramming has completely abrogated this opinion. However, to date, neuron dedifferentiation has been considered as relatively difficult. Here, it has been demonstrated that neurons can indeed change their developmental stage in a living organism while resident in their own tissue. However, when they fuse with HSPCs, they keep the memory of their neuronal identity, as these "newborn" hybrids finally differentiate into neurons. This is not a trivial observation, as researchers normally force *in vitro* reprogrammed cells to propagate with a de-differentiated phenotype; indeed, even ESCs, in principle, do not exist in the embryo. Pluripotent cells, such as the reprogrammed cells, should rapidly undergo a change of fate *in vivo,* which will depend on the different tissue signals, and they should commit to progress into a specific differentiation fate. A lineage identity memory that is not erased during the reprogramming process might be beneficial, to direct the correct differentiation path *in vivo.* Interestingly, induced pluripotent SCs (iPSCs) have been shown to retain epigenetic memory of their somatic cells of origin [Polo et al., Nat Biotechnol 28, 848-855 (2010); Kim et al., Nature 467, 285-290 (2010)]. Here, in the model used herein, the transition from one cell fate to another is not direct, but passes through the transient re-expression of precursor genes; thereby passing through an intermediate, less-differentiated, developmental precursor.

Wnt signalling controls the regeneration of tissues in response to damage in lower eukaryotes [Lengfeld et al., Dev Biol 330, 186-199 (2009)]. Regeneration of the Zebra fish tail fin and the *Xenopus* limbs requires activation of Wnt/β-catenin signalling; likewise for tissue regeneration in planarians [De Robertis, Sci Signal 3, pe21 (2010)]. Interestingly, in fish and postnatal chicken retina, down-regulation of Müller cell specific markers, such as glutamine synthetase and activation of progenitor markers, such as Pax6 and Chx10 have been associated to a regenerative potential of these cells. However, exogenous activation of Wnt signalling is necessary to induce Müller cell de-differentiation in mouse retina. The Wnt signalling regenerative activity that is present in lower eukaryotes might therefore have been lost during evolution.

Although all of these studies highlight the important role of the Wnt/β-catenin signalling pathway in the regeneration process, the biological mechanisms that form the basis of this regeneration were still largely unknown to date; here, it is shown that at least in mouse retina, regeneration can occur through cell-fusion-mediated reprogramming. Moreover, this process can be induced upon recruitment of BMCs into the eye. Interestingly, a few recruited BMCs fuse with Müller cells after damage were observed. Therefore, it might well be that the de-differentiation of Müller cells, reported previously [Osakada et al., (2007) cited *supra*] is due to fusion events with recruited BMCs.

However, although other attempts to fully regenerate damaged mouse retinas after ectopic activation of Wnt signalling in the eye have failed [Osakada et al., (2007) cited *supra*] here it has been demonstrated that in addition to the activation of Wnt signalling, cell-fusion-mediated reprogramming is also essential in the regeneration process. Thus, strategies to increase BMC recruitment to the eyes along with the activation of Wnt signalling might be therapeutically relevant to regenerate damaged retinal tissue.

All in all, it can be concluded that reprogramming is not only a way to generate iPSCs *in vitro*, but it is also a physiological mechanism *in vivo*. It might well be that reprogramming is a normal mechanism of damage repair and minor damages, like photo-damage or mechanical-damage, are repaired through this cell-fusion-mediated reprogramming. It is also possible that Wnt-mediated reprogramming is a safeguard mechanism after *in vivo* cell fusion. The hybrids that are not reprogrammed undergo apoptosis-mediated cell death. Instead, Wnt-mediated reprogrammed hybrids can survive and can proliferate.

The assays show that expression of RFP and YFP transgenes derived from the genome of the two different fusion partners were detected two weeks after cell fusion, which indicates the contribution of both genomes in the hybrids. Moreover, proliferation of the reprogrammed hybrids was observed, an indication that they were mononucleate cells or *bona-fide* synkarions. Stable heterokaryons have been seen with Purkinje cell fusion with BM-derived cells, and their numbers were greatly increased upon inflammation [Johansson et al., Nat Cell Biol 10, 575-583 (2008)]. In addition, recently, heterokaryons have been also found in wild-type retinas [Morillo et al., Proc Natl Acad Sci U S A 107, 109-114 (2010)]. However, inventors never detected heterokaryons in the retina of the injected eyes, although its presence cannot be formally excluded.

On the other hand, it should be taken into account that detrimental consequences were seen when increased resistance to apoptosis was observed after fusion of cancer SCs with somatic cells, such as multidrug resistance of a developing tumour (Lu & Kang. Cancer Res 69, 8536-8539 (2009)]. Moreover, cell fusion, and thus polyploid cells, can also arise during pathological conditions, and often the genetic instability in these cells can lead to aneuploidy and the development of cancers. Thus, data provided by the present invention might also be important in the future to follow a different path; i.e., to study the fusion of cancer SCs with somatic cells during tumour development.

Reprogramming is a gradual and slow process, with lineage specific genes silenced, while endogenous genes associated with pluripotency are induced. Overall, the process is very inefficient, because of the different genetic and epigenetic barriers [Sanges & Cosma. Int J Dev Biol 54, 1575-1587 (2010)]. Indeed pre-iPS (partially reprogrammed cells) show incomplete epigenetic remodelling and persistent DNA hypermethylation, among other features. They can be converted into iPS cells through global inhibition of DNA methylation. Inventors recently showed that deletion of Tcf3, which is a repressor of β-catenin target genes, relieves epigenome modifications during reprogramming, thereby facilitating iPS cell derivation *in vitro* [Lluis et al., (2011) in press]. This might also take place during *in vivo* cell fusion: the epigenome of SPCs might be actively remodelled, and some reprogrammers transcribed, which might change the neuronal epigenome in the hybrids *in trans*.

In conclusion, it can be asserted that cell-fusion-mediated reprogramming controlled by Wnt signalling is a physiological *in vivo* process, which can contribute to cell regeneration/repair in normal tissues.

## Claims

1. A pluripotent cell selected from the group consisting of:
(i) a pluripotent cell treated with a Wnt/β-catenin pathway activator, and
(ii) a pluripotent cell that overexpresses a Wnt/β-catenin pathway activator, for use in the treatment of a retinal degeneration disease.

2. Pluripotent cell for use according to claim 1, wherein the treatment of the retinal degeneration disease is by reprogramming of retinal neurons mediated by cell fusion of said pluripotent cell with said retinal neurons, said reprogramming being mediated by activation of the Wnt/β-catenin pathway.

3. Pluripotent cell for use according to claim 1, wherein said Wnt/β-catenin pathway activator is selected from the group comprising β-catenin, a Wnt isoform, and a glycogen synthase kinase-3 (GSK-3) inhibitor.

4. A pluripotent cell for use in the treatment of a retinal degeneration disease, by reprogramming of retinal neurons mediated by cell fusion of said pluripotent cell with said retinal neurons, said reprogramming being mediated by activation of the Wnt/β-catenin pathway.

5. Pluripotent cell for use according to claim 1 or 4, wherein said pluripotent cell is an adipose-derived stem cell (ASC), an amniotic stem cell, a bone marrow-derived stem cell (BMSC), a cord blood-derived stem cell (CBSC), an embryonic stem cell (ESC), an endothelial stem cell, an epidermal stem cell, a haematopoietic stem and progenitor cell (HSPC), a mesenchymal stem cell (MSC), a neural stem cell (NSC), or a retinal stem cell (RSPC); preferably an ESC, a HSPC or a RSPC.

6. Pluripotent cell for use according to claim 1 or 4, wherein said retinal degeneration disease is selected from the group consisting of retinitis pigmentosa, age-related macular degeneration, Stargardt disease, cone-rod dystrophy, congenital stationary night blindness, Leber congenital amaurosis, Best's vitelliform macular dystrophy, anterior ischemic optic neuropathy, choroideremia, cone-rod dystrophy, age-related macular degeneration, foveomacular dystrophy, Bietti crystalline corneoretinal dystrophy, Usher's syndrome and a retinal degenerative condition derived from a primary pathology.

7. A cell composition, wherein at least 50% of the cells of said cell composition are pluripotent cells according to any of claims 1 to 3 or 5.

8. A pharmaceutical composition comprising at least a pluripotent cell according to any of claims 1 to 3 or 5, and a pharmaceutically acceptable carrier.

9. A kit comprising a pluripotent cell according to any of claims 1 to 3 or 5, and instructions for use of the kit components.

10. Use of the kit of claim 9 in the treatment of a retinal degeneration disease.
